# EUROPEAN PATENT APPLICATION

(11) **EP 4 220 282 A1**
(43) Date of publication of application: **02.08.2023**
(21) Application number: 22153835.8
(22) Date of filing: 28.01.2022
(51) Int. Cl.: G02C 7/02, A61B 3/028

(54) **DETERMINING A LENS SHAPE FOR PRODUCING AN OPTICAL LENS FOR AN EYE OF A PERSON**

(71) Applicant: Carl Zeiss Vision International GmbH, 73430 Aalen (DE)
(72) Inventor: Leube, Alexander, 73434 Aalen (DE); Nehrbass, Eric, 73431 Aalen (DE); Jester, Philipp, 89520 Heidenheim an der Brenz (DE); Habtegiorgis, Selam Wondimu, 73431 Aalen (DE)
(74) Representative: Altmann Stößel Dick Patentanwälte PartG mbB

(57) **Abstract**

The present invention relates to a computer-implemented method (400), a computer program and an apparatus (100) for determining at least one lens shape (202) for producing at least one optical lens (200) for at least one eye (302) of a person (300) as well as to use of a personal computer. Herein, the method (400) comprises the following steps:
a) receiving first information (450) about at least one refractive error of at least one eye (302) of a person (300) and second information (440) related to the person (300);
b) determining at least one lens shape (202) for at least one optical lens (200) for the at least one eye (302) of the person (300) designated for correcting the at least one refractive error of the at least one eye (302) of the person (300) using the first information (450), wherein at least one lens shape parameter of the at least one optical lens (200) is adapted to the second information (440) related to the person (300),
wherein at least one piece of the second information (440) is determined from data recorded by at least one electronic component (102) of the at least one personal computer used by the person (300).

As the present invention relies on recording the second information (440) using a personal computer, the recording may be performed anywhere and by any person.

## Description

### Field of the invention

The present invention relates to a computer-implemented method, a computer program, and an apparatus for determining at least one lens shape for producing at least one optical lens for at least one eye of a person. It further relates to a use of a personal computer in a method for determining at least one lens shape for producing at least one optical lens for at least one eye of a person. Herein, the at least one optical lens may, in particular be, selected from a spectacle lens, a contact lens, or an intraocular lens.

### Related art

Sumit Majumder and M. Jamal Deen, Smartphone Sensors for Health Monitoring and Diagnosis, Sensors, 2019, 19, 2164 describes that over the past few decades a dramatic rise in life expectancy was witnessed owing to significant advances in medical science and technology, medicine as well as increased awareness about nutrition, education, and environmental and personal hygiene. Consequently, the elderly population in many countries are expected to rise rapidly in the coming years. A rapidly rising elderly demographics is expected to adversely affect the socioeconomic systems of many nations in terms of costs associated with their healthcare and wellbeing. In addition, diseases related to the cardiovascular system, eye, respiratory system, skin and mental health are widespread globally. However, most of these diseases can be avoided and/or properly managed through continuous monitoring. In order to enable continuous health monitoring as well as to serve growing healthcare needs; affordable, non-invasive and easy-to-use healthcare solutions are critical. The ever-increasing penetration of smartphones, coupled with embedded sensors and modern communication technologies, make it an attractive technology for enabling continuous and remote monitoring of an individual's health and wellbeing with negligible additional costs. A comprehensive review is presented of the state-of-the-art research and developments in smartphone-sensor based healthcare technologies.

Laura Boccardo, Viewing distance of smartphones in presbyopic and non-presbyopic age, Journal of Optometry, 14, (2021), 120 -126 describes that the aim of a cross-sectional observational study was to measure habitual viewing distance with smartphones in individuals of different ages, and to identify factors influencing viewing distance. Significant differences between males and females were observed, due to the different average body size between the two gender groups. Average viewing distance with smartphones in presbyopes matched approximately to the typical near reading distance of 40 cm. In a non-presbyopic group, the accommodative demand when reading a smartphone was slightly higher than in the presbyopic group. High variability was observed in both age groups, without a statistically significant correlation with other assessed factors as reading position, ametropia, correction modality, and near vision quality.

US 2019/0246896 A1 discloses a measurement method for determining a value of a visual correction need for near vision of an individual in a natural posture for near vision. A recognizing limit distance is determined so that the individual is able to recognize at least one predetermined symbol farther than the limit distance relatively to the individual and unable to recognize the at least one predetermined symbol closer to the individual than the limit distance. A portable medium displaying the predetermined symbol is displaced in front of the face and eyes of the individual to change a frontal distance between his/her eyes and the portable medium and in which when a recognizing limit distance is detected by the individual, the limit distance is measured and the value of the visual correction need is determined from the measured limit distance, wherein the visual correction need includes an accommodation compensation need.

WO 03/052491 A1 involves the prescribing and/or dispensing ophthalmic lenses, such as progressive addition lenses, for a wearer. In one form of the invention lens usage information is obtained from a wearer and entered into a programmed computer. The programmed computer processes the lens usage information to provide a separate weighted lifestyle score for each of one or more respective lifestyle score categories, such that each weighted lifestyle score is a function of a predetermined relationship between the respective lifestyle score category and at least ophthalmic lens design feature. The programmed computer then selects or designs an ophthalmic lens design using one or more of the weighted lifestyle scores such that the selected or designed ophthalmic lens has at least one lens design feature which has been customized using one or more of the weighted lifestyle scores.

EP 3 730 038 A1 discloses a system and method for interactively measuring ocular refractive errors, addition and power of reading glasses without the need of an optical component that would change optical vergence of a target. The system can measure the distance from a user's eyes to either or both border of interval of clear vision, for one orientation or two perpendicular orientations. The measurements together with the user's age can be used to estimate sphero-cylindrical refraction, addition and power of reading glasses. The system can use different sizes, directions and colors of targets which can change with said distance or user interaction.

EP 3 381 351 A1 relates to a system for assessing a health condition of a user comprises a sensor unit, a monitoring unit and a storage unit. The sensor unit comprises at least one eye sensor. The at least one eye sensor is adapted to obtain an optical signal reflected from an eye and/or surrounding tissues of the user. The sensor unit can be mounted on a wearable device. The monitoring unit is connected to the sensor unit. The monitoring unit is adapted to derive data related to an eye activity of the user by processing the optical signal. The data related to the eye activity of the user is included in the optical signal. The storage unit is connected to the monitoring unit. The storage unit is adapted to store the derived data related to the eye activity of the user and recorded data. The monitoring unit is further adapted to obtain the recorded data from the storage unit. The monitoring unit is further adapted to assess the health condition of the user by comparing the recorded data with the derived data related to the eye activity of the user. Further, the invention relates to a method for assessing the health condition of the user.

EP 3 569 137 A1 relates to an apparatus for customizing an optical lens which comprises an observation unit adapted to acquire at least one of visual activities of a user and viewing distance profiles of the visual activities, a processor adapted to calculate a personal distance profile based on at least one of the acquired visual activities and the acquired viewing distance profiles, and an implementation unit adapted to customize the optical lens based on at least one of the acquired visual activities and the acquired personal distance profile.

### Problem to be solved

It is therefore an objective of the present invention, in particular in view of EP 3 569 137 A1, to provide a computer-implemented method, a computer program, an apparatus and a use of a personal computer in a method for determining at least one lens shape for producing at least one optical lens for at least one eye of a person, which at least partially overcome the above-mentioned problems of the state of the art.

It is a particular objective of the present invention to provide a reliable, easy accessible, cheap and convenient approach for recording and maintaining individual data used to determine the least one lens shape, which can be performed anywhere and by any person, including but not limited to in less-developed countries.

### Summary of the invention

This problem is solved by a computer-implemented method, a computer program, and an apparatus for determining at least one lens shape for producing at least one optical lens for at least one eye of a person and a use of a personal computer, which at least partially overcome the above-mentioned problems of the state of the art with the features of the independent claims. Preferred embodiments, which might be implemented in an isolated fashion or in any arbitrary combination, are listed in the dependent claims or throughout the following description.

In a first aspect, the present invention relates to a computer-implemented method for determining at least one lens shape for producing at least one optical lens for at least one eye of a person, the method comprising the following steps:
a) receiving first information about at least one refractive error of at least one eye of a person and second information related to the person; and
b) determining at least one lens shape for at least one optical lens for the at least one eye of a person designated for correcting the at least one refractive error of the at least one eye of the person using the first information, wherein at least one lens shape parameter of the at least one optical lens is adapted to the second information related to the person;
wherein at least one piece of the second information is determined from data recorded by at least one electronic component of at least one personal computer used by the person.

Herein, the indicated steps may, preferably, be performed in the given order, commencing with step a) and finishing with step b). However, any or all of the indicated steps may also be repeated several times and/or preformed concurrently in part.

As generally used, the term "computer-implemented method" refers to a method which involves a programmable apparatus, in particular, a computer, a computer network, a readable medium carrying a program, or a processing device of at least one personal computer, whereby at least one of the steps of the method, specifically at least one of steps b) and c), are performed by using at least one computer program. Alternatively, the at least one computer program may be accessible by an apparatus which may be adapted for performing the method via a network, such as via an in-house network or via internet. With particular regard to the present invention, the present method can, thus, being performed on a programmable apparatus, specifically on the processing device of the at least one personal computer, which is configured for this purpose, such as by providing a computer program which is configured for such a purpose.

As generally used, the term "determining" or any grammatical variation thereof refers to a process of generating representative results which are, typically, denoted as "data". With particular regard to the present invention, the data comprise information about the lens shape for producing at least one optical lens for at least one eye of a person.

As used herein, the term "lens shape" refers to a form of an optical lens, particularly the three-dimensional appearance of the optical lens. Further, the lens shape can, additionally, be determined by at least one parameter which is not directly related to the form. Such a parameter may, specifically, be selected from a material of the optical lens and/or a refractive index of the material of the optical lens.

As generally used, the term "producing" or any grammatical variation thereof refers to designing a geometrical model of at least one optical lens for at least one eye of a person, and/or to manufacturing the at least one optical lens for the at least one eye of the person. The geometrical model of the optical lens comprises geometrical data of the at least one optical lens, particularly comprises exclusively the geometrical data of the at least one optical lens. The geometrical data may be at least one radius of at least one curvature of the at least one optical lens. In the process of manufacturing the at least one optical lens, a hard copy of the at least one optical lens is, eventually, made available to the person. Instead of the term "person", a different term, such as "subject", or "wearer", may also be applicable.

According to step a), both first information about at least one refractive error of at least one eye of a person and second information related to the person is received, wherein the first information and the second information are made available to computer-implemented method.

As used herein, the term "receiving" or any grammatical variation thereof refers to making information available to a computer-implemented method, especially for being processed by at least one further step as comprised by the computer-implemented method, in particular for being used as an input for an algorithm as, subsequently, employed by the computer-implemented method.

As used herein, the term "information" refers to a context of data. For this purpose, the information is comprised in the data. As used herein, the terms "first" or "second" are considered as a description of an element without specifying an order or a chronological sequence and without excluding a possibility that other elements of the same may be present. Herein, the "first" element is different from the "second" element.

As used herein, the term "second information" refers to an information that is different from the first information and that influences the at least one lens shape. The second information may be about at least one further body feature of the person, more particularly of the at least one eye of the person, more particularly of the at least one eye of the person; at least one habit of the person, particularly at least one habit related to the use of the at least one personal computer, or particularly at least one habit related to an eye movement and/or head movement characteristic of the person; or the environment of the person.

As generally used, the terms "refraction" or "refractive" refer to a bending of incident light entering the interior of the eye of the person via the pupil, wherein the term "refractive error" refers to an observation that the incident light may, in particular owing to a form of the eye, not be focusing appropriately on the retina of the eye, resulting in a defocus of the eye. The first information about the at least one refractive error corresponds to at least one optical parameter of the optical lens to be produced. Based on standard ISO 13666:2019, also herein referred to as "Standard", the term "optical parameter" relates to at least one property of the at least one optical lens, preferably of at least one spectacle lens, which is capable of altering the incident light beam through the at least one optical lens, preferably the at least one spectacle lens. As indicated above, the Standard, particularly Sections 3.10, 3.12 and 3.13, deals with optical parameters of spectacle lenses, in particular with regard to spherical and astigmatic properties of different kinds of spectacle lenses.

According to step b), the at least one lens shape for the at least one optical lens for the at least one eye of the person designated for correcting the at least one refractive error of the at least one eye of the person is determined. In other words, the lens shape for the at least one optical lens is determined by determining at least one value for the at least one optical parameter which is appropriate for compensating the at least one refractive error. However, in general, there still remains at least one degree of freedom, preferably a multitude of degrees of freedom, with regard to the specific design of the lens shape, in which manner at least one lens shape parameter can be further adapted.

Further according to step b), at least one lens shape parameter of the at least one optical lens is adapted to the second information related to the person. As indicated above, the lens shape can be determined by taking into account the at least one degree of freedom with regard to the specific design of the lens shape. As used herein, the term "lens shape parameter" refers to at least one value which participates in constituting the lens shape, wherein the at least one lens shape parameter may be chosen by taking into account the degrees of freedom. As used herein, the term "adapting" or any grammatical variation thereof refers to aligning the at least one lens shape parameter to the second information by, further, taking into account the first information. As a consequence, the lens shape is determined by the first information and the second information.

In accordance with the present invention, at least one piece of the second information is determined from data recorded by at least one electronic component of at least one personal computer used by the person. As general used, the term "piece of the second information" refers to a context within the second information. The second information may comprise only the context given by the at least one piece of the second information or, in addition, at least one further context. The piece of the second information may, thus, be a portion or an item of the second information or, alternatively, the piece the second of information may be the second information. The piece of the second information may, thus, comprise a portion of the data of the second information or may comprise all the data of the second information. As generally used, the term "data" refers to an entity of information, such as a flag, a numeric or an alphanumeric value, particularly an entity that may be readable and/or processable by a computer which comprises at least one piece of the first information and/or the second information, particularly the first information and/or the second information. As further generally used, the term "recording" or any grammatical variation thereof refers to a capturing of at least one piece, preferably a plurality of pieces, of data. The data is made available to the computer-implemented method for determining at least one lens shape for producing at least one optical lens for at least one eye of a person.

As used herein, the term "electronic component" refers to a device which is completely comprised by the at least one personal computer and connected to the at least one processing device of the at least one personal computer, wherein the at least one electronic component is designated for recording data comprising the at least one piece of the second information. Preferably, a complete physical connection, in particular a wire-bound or a wireless connection which is designated for transmitting at least one of: an electrical current, an electric voltage, or an optical signal between the at least one electronic component and the at least one processing device, may be used for this purpose.

As generally used, the term "personal computer" refers to a multi-purpose computer whose size, capabilities, and price make it feasible for an individual use. Personal computers are configured for being operated directly by an end user, rather than by a computer expert or a technician. In the context of the present invention, the personal computer is used by the person, for which the at least one optical lens is going to be produced, for recording the at least one piece of the second information. The personal computer may, further, be used for at least one software application. Such a software application may be a web browser or a word processor program.

In a preferred embodiment, the at least one personal computer may be selected from at least one of:
- a smartphone;
- a smart watch;
- a smart glasses;
- a virtual reality head set;
- a desktop computer;
- a laptop; or
- a tablet,
wherein the smartphone may, particularly, be preferred, specifically owing to both a high availability in practice and a high practical suitability for the present invention.

As generally used, the term "smartphone" refers to a mobile phone having computer functionalities and connectivity and may, additionally, comprise at least one sensor and/or at least one actuator, for example at least one vibration motor. As generally used, the term "smart watch" refers to an electronic wristwatch that has computer functionalities and connectivity and may, additionally, comprise at least one sensor and/or at least one actuator, for example at least one vibration motor. As generally used, the term "smart glasses" refers to wearable spectacles having computer functionalities and connectivity, and may, additionally, add information onto at least one optical lens alongside to what the wearer sees. As further generally used, the term "virtual reality head set" is a head-mounted display designed to give a wearer access to a virtual reality. As generally used, the term "desktop computer" refers to a computer in a housing shape suitable for use as a workstation computer on desks. As generally used, the term "tablet" refers to a portable, flat touch-screen computer. As generally used, the term "laptop" refers to a special type of computer having a screen being movably attached to a housing, wherein the screen can be folded onto the housing.

The term "optical lens" refers to a visual aid which is used for determining and/or correcting a defective vision of a wearer of the optical lens. In accordance with the present invention, the wearer of the at least one spectacle lens is the person for whom the related second information is determined. For determining and/or correcting the defective vision of the wearer of the optical lens, the optical lens has a lens body which comprises a particular lens shape which is configured for this purpose and which is determined according to the present invention.

In a preferred embodiment, the at least one optical lens is selected from at least one of:
- a spectacle lens;
- a contact lens; or
- an intraocular lens.

Based on the "Standard", 3.5.2, the term "spectacle lens" refers to an optical lens which is used for determining and/or correcting a defective vision of a wearer of the optical lens, wherein the optical lens is carried in front of the eye of the person, thereby avoiding a direct contact with the eye of a wearer. As generally used, the term "contact lens" refers to a lens placed directly on the surface of the eye of the wearer to correct visual defects. As further generally used, the term, "intraocular lens" refers to an artificial lens implanted in the eye of a wearer for correcting the defective vision.

In a preferred embodiment, the at least one spectacle lens may be selected from:
- a single vision lens;
- a bifocal lens;
- a multifocal lens;
- a variofocal lens; or
- a progressive-power lens.

Based on the "Standard", particularly in the Sections 3.7.1, 3-4, the term "single-vision lens" refers to a particular type of spectacle lens having a single dioptric power, while the terms "bifocal lens" and "multifocal lens" refer to a particular type of spectacle lens comprising two or more portions, respectively, wherein each portion has a different value for the dioptric power. Based on Section 3.7.8 of the "Standard", the term "progressive-power lens" and the term "variofocal lens" refer to a power-variation lens having two reference points for focal power, generally designed to provide correction for presbyopia and clear vision from distance to near. Specifically, a progressive-power lens has a primary reference point that is a distance reference point and a secondary reference point that is a near reference point.

In a preferred embodiment, the at least one refractive error of the at least one eye of the person may, specifically, be at least one of a value related to:
- a spherical power;
- a cylinder;
- a cylinder axis;
- a prism;
- a prism base setting; or
- an addition.

As defined in the Standard, Section 3.12.2, the term "spherical power", usually abbreviated to "sphere" or "sph", refers to a value of a back vertex power of a spherical-power lens, or for a back vertex power in one of two principal meridians of an astigmatic-power lens, depending on a principal meridian chosen for reference. As defined in the Standard, Section 3.13.7, the term "cylinder", usually abbreviated to "cylinder" or "cyl", refers to an algebraic difference between principal powers with power of the principal meridian chosen for reference being subtracted from the other principal power. As defined in the Standard, Section 3.13.8, the term "cylinder axis", usually abbreviated to "cyl axis" or "axis", refers to a direction of the principal meridian of a lens whose vertex power is chosen for reference. As defined in the Standard, Section 3.11.10, the term "prismatic power", usually abbreviated to "prism" refers to a magnitude of a prismatic effect which is a collective name for a prismatic deviation and a prism base setting, wherein the term "prismatic deviation" indicates a change in direction imposed on a ray of light as a result of refraction. As defined in the Standard, Section 3.11.7, the term "prism base setting", usually abbreviated to "base" refers to a direction of a line from an apex to a base in a principal section of a prism. As defined in the Standard, Section 3.16.3, the term "addition", also abbreviated to "add", refers to a difference between the vertex power of a near portion and the vertex power of a distance portion in a multifocal or power-variation lens.

In a preferred embodiment, the at least one refractive error of the at least one eye of the person may, particularly, be received from at least one of:
- determining by using the at least one personal computer;
- determining by using at least one lensometer;
- determining by using at least one autorefractor;
- determining by using at least one retinoscope;
- reading in at least one optical lens prescription document;
- determining by using at least one subjective refraction workflow.

As generally used, the term "lensometer" refers to an ophthalmic instrument, also known as focimeter or vertometer. A lensometer is mainly used by an optometrist and/or an optician to determine at least one optical parameter of a spectacle lens, particularly of a spectacle lens used by the person to correct the refractive error of the at least one eye of the person. As further generally used, the term "autorefractor" refers to a computer-controlled machine used during an eye examination by the optometrist and/or the optician providing an objective measurement of the refractive error of the person. As further generally used, the term "retinoscope" refers to a device used to shine light into at least one eye of a patient, particularly the person, and to observe a reflection reflex off the patient's retina for determining the refractive error of the patient. As further generally used, the "lens prescription document" refers to a form containing information about an optical lens particularly information at least one optical parameter, used by the person to correct the refractive error of the at least one eye of the person. As further generally used, the term "subjective refraction workflow" refers to a technique of the optometrist and/or the optician to determine, the combination of spectacle lenses providing the best corrected visual acuity. Thereby the at least one refractive error is determined.

In a preferred embodiment, the second information may comprise at least one piece of second information which may be selected from at least one of:
- at least one pupil size of the at least one eye of the person;
- at least one direction of a line of sight of the at least one eye of the person;
- at least one angle between a primary direction and the line of sight of the person;
- at least one orientation of the head of the person;
at least one angle of inclination. As generally used, the term "indoors" refers to the person being within a building, while the term "outdoors" refers to the person being outside of the building. As further generally used, the term "ultraviolet index" refers to is an international standard measurement of a strength of ultraviolet radiation. As further generally used, the term "electromagnetic spectrum" refers to a range of frequencies of electromagnetic radiation and respective wavelengths and photon energies, in particular the visual spectral range and adjacent spectral ranges.

In a preferred embodiment, the at least one piece of the second information, particularly a plurality of pieces of the second information, recorded in a predefined time interval can be assigned to an event. As used herein, the term "plurality" refers to a quantity of at least two units, preferably more than two units, particularly a quantity of least two pieces, preferably of more than two pieces, of the second information. As used herein, the term "event" refers to data which satisfy a predefined criterion. The data may be at least one piece of the second information and/or a plurality of pieces of the second information. The criterion may, preferably, be that the data was recorded within a predefined time interval. However, a further kind of criterion may also be feasible.

In a preferred embodiment, the at least one electronic component of the at least one personal computer may be selected from least one of:
- at least one image capturing device;
- at least one geopositioning sensor;
- at least one distance sensor;
- at least one depth sensor;
- at least one ambient light level sensor;
- at least one screen inclination sensor; or
- at least one storage device.

As generally used, the term "image capturing device" refers to a technical component comprising an at least one image sensor for capturing at least one image. As generally used, the term "sensor", also known as detector, refers to a technical component designated for measuring at least one physical and/or chemical property qualitatively and/or quantitatively. As used herein, the term "geoposition" or any geometrical variations thereof, refers to a location. The location may be on the earth. As generally used, the term "storage device" refers to an electronic data storage unit for recording of data comprising information. As generally used, the term "distance sensor" refers to a distance measuring device. The distance measuring device may be at least one image capturing device, particularly a camera of a smart phone, more particularly a back and/or a front camera of the smartphone. For measuring the distance, the distance measuring device, particularly the at least one image capturing device, more particularly the respective camera, may be calibrated using standard calibration methods. As generally used, the term "depth sensor" refers to a sensor for acquiring a distance information for at least one point, particularly for a multitude of points. The depth sensor, thereby, offers a field of view in which the distance information is acquired. The depth sensor may rely on time measurements, particularly on measurements of a time difference between emitting of an electromagnetic wave by the sensor and detecting of the reflected electromagnetic wave by the sensor.

In a preferred embodiment, the at least one image capturing device may be selected from at least one of:
- at least one camera; particularly a front camera and/or back camera of a smartphone;
- at least one smart sensor, particularly a combination of a plurality of smart sensors; or
- at least one webcam.

As generally used, the term "camera" refers to an optical device that captures visual images. As generally used, the term "smart sensor" refers to a portable electronic detection device having a connectivity. As generally used, the term "webcam" refers to a small camera that may be placed on a monitor, or may be introduced into a computer.

In a preferred embodiment, the at least one geopositioning sensor may be a tracking unit of at least one of:
- the Global Positioning System, abbreviated GPS;
- the Galileo Satellite Navigation;
- the Global Navigation Satellite System, abbreviated GNSS;
- the BeiDou Navigation Satellite System; or
- the Indian Regional Navigation Satellite System, abbreviated IRNSS.

As generally used, the term "tracking unit" refers to device interacting with the respective positioning system for determining a position of a location of the person. As generally used, the term "Global Positioning System" refers to a satellite-based radio navigation system owned by the United States government. As generally used, the term "Galileo Satellite Navigation" refers to a global navigation satellite system established by the European Union. As generally used, the term "Global Navigation Satellite System " refers to a Russian space-based satellite navigation system operating. The As generally used, the term "BeiDou Navigation Satellite System" refers to a Chinese satellite navigation system. As generally used, the term "Indian Regional Navigation Satellite System" refers to an Indian autonomous regional satellite navigation system.

In a preferred embodiment, the at least one storage device may be selected from least one of:
- at least one hard disk;
- at least one solid state drive; or
- at least one random access memory.

As generally used, the term "hard disk", also known as "hard disk drive" or "fixed disk", refers to an electro-mechanical data storage device that stores and retrieves data using magnetic storage and at least one rotating plate coated with a magnetic material. As further generally used, the term "solid state drive" refers to a storage device that uses integrated circuit assemblies to store data persistently. As further generally used, the term "random access memory" refers to a form of computer memory that can be read and changed in any order, typically used to store working data and machine code.

In a preferred embodiment, the at least one screen inclination sensor may be selected from at least one of:
- at least one accelerometer; or
- at least one gyroscope.

As generally used, the term "accelerometer" refers to a device measuring its acceleration, typically by determining the inertial force acting on a test mass. As further generally used, the term "gyroscope" refers to a device used for measuring or maintaining orientation and angular velocity. Typically, a gyroscope comprises a spinning wheel and/or a spinning disc within a mounting, wherein the spinning wheel and/or the spinning disc is unaffected by tilting and/or rotation of the mounting.

In a preferred embodiment, the piece of the second information comprised in data recorded by the at least one image capturing device may comprise at least one of:
- the at least one pupil size of the at least one eye of the person.
- the at least one direction of a line of sight of the at least one eye of the person;
- the at least one angle between a primary direction and the line of sight of the person;
- the at least one orientation of the head of the person;
- the at least one eye movement characteristics of the person;
- at least one electromagnetic spectrum prevailing at the location of the person;
- at least one ultraviolet index at the location of the person; or
- at least one infrared index at the location of the person;
- the at least one ambient light level at the location of the person; or
- at least one distance between the at least one eye of the person and at least one object located at line of sight of the person.

The respective data may be when the person is using the at least one personal computer.

In a preferred embodiment, the at least one image capturing device may record at least one image, particularly of the person, more particularly of the at least one eye of the person, at at least one point of time that is unknown to the person and/or at at least one point of time that is known to the at least one person. As used herein, the term "unknown" indicates a situation in which the person is unaware of the capturing device recording the at least one image. In a preferred embodiment, the data recorded by the at least one image capturing device may be at least one image of the person that shows the person in a natural habitus. As generally used, the term "natural habitus" refers to an unobserved, natural, relaxed, and comfortable posture of the head, of the eye and of the body which the person, typically, assumes an absence of psychological stress.

In a preferred embodiment, the data recorded by the at least one image capturing device may be at least one image of the person that shows the person in a predefined habitus. As generally used, the term "predefined habitus" refers to a habitus that the person may adopt when using the at least one personal computer, particularly when using the smartphone. In a preferred embodiment, at least one instruction may be given to the person, particularly by displaying the at least one instruction on a screen of the at least one personal computer, on how to adopt the predefined habitus.

In a preferred embodiment, the data recorded by the at least one image capturing device may be at least one image of the person that shows the person wearing spectacles; however at least one image in which the person may not be wearing spectacles may also be feasible.

In a preferred embodiment, the data recorded by the at least one image capturing device may be an image of the person which may be processed by using at least one of:
- at least one image processing method; or
- at least one machine learning based method.

As generally used, an "image processing method" is a computer based method that, in particular, performs image segmentation. The term "machine learning based method" is a method that comprises at least one machine learning algorithm, particularly with regard to image processing. As generally used, the term "machine learning algorithm" or "machine learning model" refers to a process of applying artificial intelligence to automatically generate a statistical model. A machine learning algorithm configured to generate the desired statistical model which may be based on a large number of training data sets can, preferably, be used. As generally used, the term "training" or any grammatical variation thereof refers to a process of generating a trained model, in particular by determining parameters, in particular weighs, as comprised by the model. The training may comprise at least one optimization or tuning process, in which manner an optimized parameter combination may be determined.

In a preferred embodiment, the piece of second information received from data recorded by the at least one geopositioning sensor may comprise at least one item of information about at least one position of the location of the person, particularly an indication about the at least one person being indoors or outdoors. As defined above, the term "indoors" refers to the person being within a building, while the term "outdoors" refers to the person being outside of the building. In particular, a time spent indoors may refer to an activity of the person which may be performed by the person inside the building, especially during work, whereas a time spent outdoors may refer to a activity of the person which may be performed by the person outside the building in open air, especially during or after work, on weekends, or during vacations. Furthermore, an indication about the at least one person being indoors or outdoors may be provided by using a combination of at least two pieces of the second information, particularly at least two pieces of the second information received from at least two individual electronic components, e. g from the at least one geopositioning sensor and the at least one ambient light sensor.

In a preferred embodiment, the piece of second information received from data recorded by the at least one storage device may be a personal preference of the person using the at least one personal computer, particularly a personal preference of the person using the at least one personal computer selected from at least of:
- at least one brightness setting of the at least one screen of the at least one personal computer;
- at least one font size setting of the at least one person using the at least one personal computer; or
- at least one screen time of the at least one person using the at least one personal computer.

As used herein, the term "personal preference" refers to a specific liking of the person, particularly a specific liking showing an individual user setting of the person on the at least one personal computer. As generally used, the term "brightness setting" refers to an intensity of radiation which is generated by a screen of the at least one personal computer, wherein a grade of the intensity is, generally, determined by the individual user setting with regard to the intensity of the screen of the at least one personal computer. As further generally used, the term "font size" refers to a magnitude of characters as displayed on the at least one screen of the at least one personal computer.

In a preferred embodiment, the at least one font size setting corresponds to a setting of at least of:
- at least one web browser used by the person; or
- at least one word processor program used by the person.

As generally used, the term "web browser" refers to an application software for accessing the world wide web. As further generally used, the term "word processor program" refers to a computer program that provides word processing functions.

In a preferred embodiment, the piece of second information received from data recorded by the screen inclination sensor may comprise at least one angle of screen inclination of the at least one screen of the at least one personal computer of the at least one person, particularly with respect to the primary direction.

In a preferred embodiment, data recorded by at least one electronic component may be recorded in an acquisition process, wherein the acquisition process may comprise at least one of
- only one single event; or
- a plurality of events.

As generally used, the term "acquisition process" refers to the procedure of recording measurement data, particularly the piece of the second information.

In a preferred embodiment, each event may comprise a time stamp, wherein a maximal difference in the time stamps of the plurality of events may, preferably, be selected from at least one of:
- 1, 2, 3, 4, 5, 6, 7, 8, 9, 10, 11, 12, 13, 14, 15, 16, 17, 18, 19, 20, 21, 22, or 23 hours;
- 1, 2, 3, 4, 5, or 6 days;
- 1, 2 or 3 weeks; or
- 1, 2, 3, 4, 5, 6, 7, 8, 9, 10, 11 or 12 months.

As used herein, the term "time stamp" refers to a time value and/or time range, particularly a time value and/or time range assigned to the event.

In a further preferred embodiment, in which each event may comprise a time stamp, the time stamps of the plurality of the events may be continuously distributed. As used herein, the term "continuously distributed" refers to a difference between two time stamps of two consecutive events being for all events below a deviation threshold. The deviation threshold may be provided in a manner that each difference between two time stamps deviates from a mean value of all differences between two time stamps for a maximum of 20%; 15%; 10%; 5%; 3% or 1%.

In a preferred embodiment, a user, particularly the person, may be authenticated, particularly authenticated before the acquisition process may be started. As used herein, the term "authenticating" or any grammatical deviation thereof, refers to an process of identifying the person by the at least one personal computer. In a preferred embodiment, the person may be authenticated by at least one of:
- a face recognition procedure;
- a fingerprint analysis procedure; or
- a login procedure, particularly by requesting at least one of
   ∘ a login;
   ∘ a password; or
   ∘ an unlock pattern.

In a preferred embodiment, the user may be the person, an optician, an ophthalmologist an optic designer, an insurance agent or representative, or an optical lens manufacturer. As generally used, the term "face recognition procedure" refers to a process in which a face, particularly a face of the person, from a particular image and/or a particular video frame is matched with at least one face comprised in a database. As further generally used, the term "fingerprint analysis procedure" refers to a process of comparing two instances of friction ridge skin impressions, particularly from at least one finger, at least one toe, at least one palm of a hand and/or at least one sole of a foot, to determine whether these impressions are from the same person. As further generally used, the term "login procedure" refers to a process of registering to a computer system, particularly the at least one personal procedure. As further generally used, the term "login" refers to an identifier of a user, particularly of the person. The term "password" refers to a personal character string that is used for a login. The term "unlock pattern" refers to a figure that has to been draw on the screen for the login. In a preferred embodiment, the acquisition process may be implemented as a background process. As generally used, the term "background process" refers to a computer process that runs without an intervention or required action of the user. The user may run and interact with other processes during the background process is running. The running of the background process may not be visible on the at least one screen.

In a preferred embodiment, the second information may be complemented or modified by receiving at least one of:
- personal data of the person, particularly at least one of:
   ∘ an age;
   ∘ a gender;
   ∘ an ethnicity; or
   ∘ a profession;
- at least one item of personal preference of the person for the at least one lens shape parameter and/or a recommendation of the optician for the at least one lens shape parameter; or
- at least one piece of the second information, particularly one piece of the second information that is not recorded by the at least one electronic component of at least one personal computer used by the person.

As used herein, the term "complementing" or any grammatical variation thereof refers to adding at least one new piece of the second information, wherein the at least one new piece of the second information has not been comprised by the second information before. As further used herein, the term "modifying" or any grammatical variation thereof refers to altering a piece of the second information that is already comprised by the second information. As generally used, the term "personal data" refers to any information directly and/or indirectly related to the person. As further generally used, the term "ethnicity" refers to a grouping of people identifying with each other by shared attributes that distinguish them from other groups, such as a common set of traditions, ancestry, language, history, society, culture, nation, religion, or social treatment within their residing area. As further generally used, the term "profession" refers to an occupation, particularly founded upon specialized educational training, the purpose of which is to supply disinterested objective counsel and service to others, for a direct and definite compensation.

In a preferred embodiment, the first information and/or the second information may be at least one of:
- complemented or modified; or
- examined
   by using at least one of:
- an user interface;
- a cloud interface; or
- a connecting interface.

As used herein, the term "examining" or any grammatical variation thereof refers to investigating an information, particularly investigating the second information, particularly to draw conclusions which lead to a personal preference and/or a recommendation. As generally used, the term "user interface" refers to a boundary between a user and a computer enabling an interaction between both. The computer may be the at least one personal computer. As further generally used, the term "cloud interface" refers to a boundary between a user and a cloud server enabling an interaction between both. As further generally used, the term "connecting interface" refers to shared boundary across which two or more separate components of a computer system transfer information. Herein, a transfer may occur between at least one of: a software, a computer hardware, a peripheral device, or a user. The user may, especially, be the person, an optician, an ophthalmologist an optic designer, an insurance agent or representative, or an optical lens manufacturer.

In a preferred embodiment, the user interface and/or the cloud interface may be selected from at least one of:
- a mobile application;
- a desktop application; or
- a web application.

As generally used, the term "mobile application" refers to a computer program designed to run on at least one mobile device. As further generally used, the term "desktop applications" refers to a computer program designed to run on at least one desktop computer. As further generally used, the term "web application" refers to a computer program that runs in a web browser.

In a preferred embodiment, the user interface and/or the cloud interface can be accessed by at least one of:
- the person;
- the optician; or
- an optical lens manufacturer;
- the optic designer; or
- the insurance agent or representative.

As generally used, the term "optician" refers to a technical practitioner who designs, fits and dispenses lenses for the correction of a person's vision. As further generally used, the term "optical lens manufacturer" is a company that exclusively or predominantly designs geometrical models of optical lenses and/or manufactures optical lenses, in particular wherein the optical lenses are at least one of spectacle lenses, contact lenses, or intraocular lenses. As further generally used, the term "optic designer" is a person designing the geometrical model of the optical lenses. As further generally used, the term "insurance representative" refers to a person working for an insurance company, particularly a health insurance company, specifically a health insurance company offering support upon an occurrence of damages with respect to optical lenses.

In a preferred embodiment, the first information and/or the second information may be transferred to at least one of:
- a further at least one personal computer;
- a cloud server;
- the optician;
- the ophthalmologist;
- the optic designer;
- the insurance agent or representative; or
- the optical lens manufacturer.

As generally used, the term "transferred" refers to transmitting of data to a computer and/or a person. The data may be transmitted through a communication channel from one computer to another. Alternatively or in addition, a hard copy of the data may be generated and the hard copy may be given to a person. As further generally used, the term "cloud server" refers to a physical or virtual infrastructure component that runs at least one application, stores data and/or processes information at a remote location.

In a preferred embodiment, the optician may complement or modify the first information and/or the second information and/or may examine the first information and/or the second information for giving a recommendation for the at least one lens shape parameter. As used herein, the term "recommendation" refers to a suggestion or proposal for an optimized course of action, particularly an optimized course of action as provided by an expert. Alternatively or in addition, the optical lens manufacturer may complement or modify the first information and/or the second information and/or may examine the first information and/or the second information for giving a recommendation for the at least one lens shape parameter and/or provides a lens shape determining procedure used in determining the at least one lens shape comprising the at least one lens shape parameter. As used herein, the term "lens shape determining procedure" refers to a process of determining the lens shape comprising the at least one lens shape parameter by considering the first information and the second information. In a preferred embodiment, a lens shape determining procedure may be used in determining the at least one lens shape from at least the first information and the second information, wherein the at least one lens shape comprises the at least one lens shape parameter.

In a preferred embodiment, the lens shape determining procedure may consider a third information for determining the at least one lens shape, wherein the third information may, particularly, comprise at least one piece of information selected from at least one of:
- at least one statistical parameter;
- at least one parameter determined by using a machine learning model, particularly selected from at least one of:
   ∘ a facial shape parameter;
   ∘ a centration parameter;
   ∘ a fitting parameter; or
   ∘ a further lens shape parameter.

As used herein, the term "statistical parameter" refers to a default value determined from at least one statistical evaluation. As further used herein, the term "facial shape parameter" refers to a form of the face of the person. As further used herein, the term "further lens shape parameter" may refer to a parameter designated to a frame of the optical lens. As generally used, the term "centration parameter" refers to a parameter which describes a spatial relationship between the at least one optical lens, a spectacle frame, and the person, particularly a relationship considering the first information and/or the second information. As generally used, the term "spectacle frame" refers to a mount which is designed for fixedly receiving the pair of the optical lenses. For this purpose, the spectacle frame can have a receptacle on a right side and on a left side for each optical lens. The spectacle frame may comprise a transparent or non-transparent material which may, preferably, be solid but flexible and light. As generally used, the term "fitting parameter" refers to a parameter which describes a spatial relationship between the at least one optical lens, a spectacle frame, and the person, e.g. the first information and/or the second information.

In a preferred embodiment, the at least one centration parameter may, preferably, be selected from at least one of:
- an interpupillary distance, also abbreviated to "PD", which is defined in the Standard, 3.2.28, as a distance between the centers of the pupils of the eyes of the wearer, when the eyes of the wearer are in a primary position further defined in the Standard, 3.2.25, as a direction of a line of sight, usually assumed to be the horizontal, to an object at an infinite distance measured with habitual head and body posture when looking straight ahead in unaided vision;
- a vertex distance between each optical lens and each eye of the person, wherein, according to the Standard, 3.2.40, the term "vertex distance" is defined as a horizontal distance between a back surface of the optical lens intended to be fitted nearer to the eye of the wearer and an apex of the cornea as measured with the eyes of the wearer in the primary position as defined above;

- a wear pantoscopic angle of the spectacle frame, which is, according to the Standard, 3.2.37, defined as a vertical angle between the horizontal and the perpendicular to a reference line passing through the apex of the grooves of the upper and lower rims of the spectacle frame in the vertical plane containing the primary direction as defined above; or
- a face form angle, also denoted as "wrap angle", which is, according to the Standard, 3.2.39, defined as an angle between a plane of the spectacle front and one of the right plane of the lens shape or the left plane of the lens shape.

In a preferred embodiment, the at least one fitting parameter may, preferably, be selected from at least one of:
- a fitting point, wherein the term "fitting point" refers to a point on a front surface of a spectacle lens or blank stipulated by the manufacturer for positioning the optical lens in front of the eye. This definition is based on the Standard, Section 3.2.34. Or
- a minimum fitting height, wherein the term "minimum fitting height" refers to a minimum vertical distance specified by a manufacturer between thw fitting point and the upper and/or lower spectacle lens edge, measured vertically above and/or below the intended position of the fitting point. This definition is based on the Standard, Section 3.16.12. Or
- a fitting point mounting position, wherein the term "fitting point mounting position" refers to vertical and horizontal fitting distances and directions of a centration point or a fitting point respectively from the boxed center, measured in a plane of the lens shape. This definition is based on the Standard, Section 3.2.35.

In a preferred embodiment, the lens model determining procedure may be selected from at least one of:
- at least one machine learning model;
- at least one statistical model; or
- at least one expert knowledge.

As generally used, the term "statistical model" refers to a mathematical model that comprises at least one statistical assumptions. As further generally used, the term "expert" refers to a person having above-average knowledge in a field, particularly in the field of determining a lens shape comprising the at least one lens shape parameter by considering the first information and the second information. Particularly the optician, the ophthalmologist and/or the optic designer may be considered as experts.

In a preferred embodiment, the at least one lens shape parameter of at least one optical lens may be at least one of:
- a magnitude of at least one optical parameter;
- a distribution of the at least one optical parameter;
- a gradient of the at least one optical parameter;
- at least one progression profile of the at least one optical lens;
- at least one property of at least one viewing zone of the at least one optical lens; particularly
   ∘ at least one property of a viewing zone having a first surface power designed for providing a first refracting power for distance vision; or
   ∘ at least one property of a viewing zone having a second surface power designed for providing a second refractive power corresponding to near vision; or
   ∘ at least one property of a viewing zone having a third surface power designed for providing a third refracting power for a different type of vision;
- a material of the at least one optical lens;
- a filter of the at least one optical lens;
- a color tint of the at least one optical lens; or
- a refractive index of the material of the at least one optical lens.

As generally used, the term "viewing zone" refers to a portion of the progressive surface which is designated for providing refracting power for a particularly selected kind of vision. As generally used, the term "material" refers to a specific substance or a mixture of a plurality of substances that constitutes the at least one optical lens. As generally used, the term "filter" refers to a property of the at least one optical lens to select the incident light according to a certain criteria, e.g. a wavelength, a polarization and/or a direction of incidence. A typical filter may be a polarization filter and/or an UV-filter. Based on the "Standard", 3.5.6, a "color tint" of an optical lens refers to a noticeable color, including grey, in transmission. As generally used, the term "refractive index" is also known as optical density or refractive index. The term refers to an optical material property, and may particularly be defined as a ratio of a first wavelength of light in the vacuum and a second wavelength of light in the material.

In a preferred embodiment, the at least one optical parameter may be at least one of:
- the spherical power; or
- the cylinder;
- the cylinder axis;
- the prismatic power;
- the prism base setting; or
- the addition.

As further generally used, the term "progression profile" refers to a distribution of at least one optical parameter over a surface the at least one optical lens, thereby defining a relationship between a spatial position and the at least one optical parameter. In a preferred embodiment, the progression profile may be described by at least one optical lens parameter, wherein the at least one optical lens parameter may, preferably, be selected from at least one of:
- a spatial position of at least one progression start on a surface of the at least one optical lens;
- a spatial position of at least one progression end on the surface of the at least one optical lens;
- a distance between the spatial position of the at least one progression start and the spatial position of the at least one corresponding progression end on the surface of the at least one optical lens;
- a course of the progression from the progression start to the progression end; or
- a width of a progression corridor.

In a preferred embodiment, the at least at least one progression profile may, in addition to the at least one refractive error of the at least one eye of the person, be adapted to at least one of:
- the age of the person;
- the at least one angle of inclination of a screen of the at least one personal computer;
- a zone demand profile considering different viewing zones of the person;
- a head and/or eye movement profile of the person; or
- an activity profile of the person.

As generally used, the term "profile" refers to a compilation of at least one characteristic behavior of the person, particularly by considering a rate of occurrence of a particular characteristic behavior of the person. As used herein, the term "zone demand" refers to a rate of the line of sight of the at least one eye of the person to be within a predefined viewing zone. As further generally used, the term "viewing zone" refers to a portion of the progressive surface which is designated for providing refracting power for a selected kind of vision. As further generally used, the term "activity" refers to a repeated behavior of the person. In a preferred embodiment, the activity profile of the person may be determined from at least one piece of the second information and may, in addition, be received from at least one of: the user interface, the cloud interface, or the connecting interface. In this respect, the user may be queried to at least one activity, such as by using the touchscreen for posing a query and receiving a respective input which can be used for modifying and/or supplementing at least one piece of second information.

In a preferred embodiment, the at least one property of the at least one viewing zone, particularly wherein the at least one viewing zone is selected from at least one of:
∘ a viewing zone for distance vision;
∘ a viewing zone for near vision; or
∘ a viewing zone for intermediate vision;
is adapted to at least one of:
- the at least one pupil size of the at least one eye of the person, particularly the at least one pupil size of the at least one eye of the person at a given at least one of:
   ∘ at least one ambient light level;
   ∘ the at least one distance between the at least one eye of the person and at least one object located at line of sight of the person; or
   ∘ the activity profile of the person; or
- the at least one distance between the at least one eye of the person and at least one intersection point of the line of sight of the person and the screen of the at least one personal computer.

As generally used, the terms "viewing zone for distance vision" may refer to a portion of the progressive surface of the spectacle lens, which is located around the center of the progressive surface and which is designated for providing refracting power for distance vision, specifically for providing foveal vision for on-axis viewing of the wearer of the progressive spectacle lens. As generally used, the term "viewing zone for near vision" may refer to a lower viewing zone of the spectacle lens. The lower viewing zone is located along a lower portion of a vertical meridian of the progressive surface and provides a second refracting power corresponding to near vision. As generally used, the term "viewing zone for intermediate vision" may refer to a viewing zone located between the viewing zone for near vision and the viewing zone for near vision of the spectacle lens. The viewing zone for intermediate vision provides a third refracting power corresponding to intermediate vision.

In a preferred embodiment, the at least one property of the viewing zone for distance vision may be adapted to at least one of:
- the at least one pupil size of the at least one eye of the person, particularly the at least one pupil size of the at least one eye of the person at a given at least one ambient light level; or
- the at least one pupil size of the at least one eye of the person, particularly the at least one pupil size of the at least one eye of the person at a given at least one viewing distance;
- the at least one pupil size of the at least one eye of the person, particularly the at least one pupil size of the at least one eye of the person at a given at least one activity.

In a preferred embodiment, the at least one property of the viewing zone for near vision may be adapted to at least one of:
- the at least one pupil size of the at least one eye of the person, particularly the at least one pupil size of the at least one eye of the person at a given at least one ambient light level; or
- the at least one distance between the at least one eye of the person and at least one intersection point of the line of sight of the person and the screen of the at least one personal computer.

In a preferred embodiment, the at least one property of the viewing zone for intermediate vision may be adapted to at least one of:
- the at least one pupil size of the at least one eye of the person, particularly the at least one pupil size of the at least one eye of the person at a given at least one ambient light level; or
- the at least one distance between the at least one eye of the person and at least one intersection point of the line of sight of the person and the screen of the at least one personal computer.

In a preferred embodiment,
- a dioptric power of the at least one optical lens, particularly the spectacle lens, may be determined from the first information; and
- a distribution of a surface power over a surface of the at least one optical lens, particularly the spectacle lens, may be adapted to the at least one piece of second information.

Based on the definitions in Section 3.10.1-3 of the Standard, the term "dioptric power" refers to a focusing power of the optical lens, particularly the spectacle lens, which, specifically, comprises both the focal power and the prismatic power of the optical lens, particularly the spectacle lens. As further based on Section 3.10.4 of the Standard, the term "surface power" refers to a local ability of a finished surface to alter a vergence of a bundle of rays incident at the surface, wherein the surface power is determined from at least one radius of a surface and the refractive index of the optical material used for the optical lens, particularly a progressive spectacle lens.

As exemplarily illustrated below, at least one known type of design, such as a so-denoted "hard design" or "soft design", of the at least one optical lens may, preferably, be used for determining the distribution of the surface power over the surface of the at least one spectacle lens based on at least one piece of second information. Herein, a "hard designed progressive spectacle lens design" typically concentrates the unwanted astigmatism into smaller areas of the spectacle lens surface, thereby expanding the areas of perfectly clear vision at the expense of higher levels of blur and distortion. Consequently, hard designed progressive spectacle lenses generally exhibit the following characteristics: wider distance zones, wider near zones, and higher, more rapidly increasing levels of surface astigmatism when compared to soft designed progressive spectacle lenses.

Further, a "soft designed progressive spectacle lens" typically spreads the unwanted astigmatism across larger areas of the lens surface, thereby reducing the overall magnitude of blur at the expense of narrowing the zones of perfectly clear vision. The astigmatic error may penetrate into the distance zone. Consequently, softer progressive spectacle lenses generally exhibit the following characteristics: narrower distance zones, narrower near zones, and lower, more slowly increasing levels of astigmatism when compared to hard designed progressive spectacle lenses.

In a preferred embodiment, a distribution of a surface power over a surface of the at least one optical lens may be adapted to at least one of:
- the at least one screen time of the at least one person using the at least one personal computer; or
- at least one outdoor time of the at least one person spent outdoors.

As generally used, the term "screen" refers to an electronic visual display device designated for the presentation of at least one of an image, an item, text, or a video transmitted electronically.

In a further aspect, the present invention relates to a computer program comprising instructions which, when the program is executed by a computer, cause the computer to carry out a method according to the aspect of the method or any one of the preferred method embodiments. For this purpose, a computer program may comprise instructions provided by means of a computer program code which are capable of performing any or all of the steps of the methods according to the present invention and, thus, to determine at least one lens shape for producing at least one optical lens for at least one eye of a person when implemented on a computer or a data processing device. The computer program code may, preferably, be provided or an electronic component of at least one personal computer, however, providing the computer program code on a data storage medium or a separate device such as an optical storage medium, e.g. on a compact disc, directly on a computer or data processing device, or via a network, such as via an in-house network or via internet, may also be feasible. For further details concerning the computer program, reference may be made to the methods according to the present invention as disclosed elsewhere herein.

In a further aspect, the present invention relates to an apparatus for determining at least one lens shape for producing at least one optical lens for at least one eye of a person, the apparatus comprising at least one processing device configured for
∘ receiving first information about at least one refractive error of at least one eye of a person and second information related to a person; and
∘ determining at least one lens shape for least one optical lens for the at least one eye of a person designated for correcting the at least one refractive error of the at least one eye of the person using the first information, wherein at least one lens shape parameter of the at least one optical lens is adapted to the second information related to the person;
wherein the at least one processing device is further configured for determining at least one piece of the second information from data recorded by at least one electronic component of at least one personal computer used by the person.

In a preferred embodiment, the at least one processing device may further be configured for carrying out the method according to the aspect of a computer-implemented method for determining at least one lens shape for producing at least one optical lens for at least one eye of a person or any preferred embodiment any. In a preferred embodiment, the apparatus is the at least one personal computer used by the person.

In a preferred embodiment, the apparatus may be at least one of:
- a smartphone;
- a smart watch;
- a desktop computer;
- a laptop; or
- a tablet,
wherein the smartphone may, particularly, be preferred, specifically owing to both a high availability in practice and a high practical suitability for the present invention.

In a preferred embodiment, the apparatus may further comprise at least one image capturing device configured for recording the at least one piece of the second information. In a preferred embodiment, the at least one image capturing device may be at least one of:
- a camera; particularly a front camera and/or back camera of a smartphone; or
- a webcam.

In a preferred embodiment, the apparatus may further comprise at least one geopositioning sensor configured for recording the at least one piece of the second information. In a preferred embodiment, the at least one geopositioning sensor may be a tracking unit of at least one of:
- the Global Positioning System;
- the Galileo Satellite Navigation;
- the Global Navigation Satellite System;
- the BeiDou Navigation Satellite System; or
- the Indian Regional Navigation Satellite System.

In a preferred embodiment, the apparatus may further comprise at least one screen inclination sensor configured for recording the at least one piece of the second information. In a preferred embodiment, the at least one screen inclination sensor may be at least one of:
- an accelerometer; or
- a gyroscope.

In a preferred embodiment, the apparatus may further comprise at least one storage device configured for storing the at least one piece of the second information. In a preferred embodiment, the at least one storage device may be at least one of:
- at least one hard disk;
- at least one solid state drive; or
- a t least one random access memory.

In a preferred embodiment, the apparatus may further comprise at least one screen configured for displaying a "user interface enabling an interaction between a user, in particular the person, and a computer, in particular the at least one personal computer used by the person. In a preferred embodiment, the at least one screen may be at least one of:
- a touchscreen;
- a monitor; or
- a projector.

As generally used, the term "monitor" refers to an electrically controlled display for a visual displaying of information such as an image or an item. As further generally used, the term "touchscreen" refers to device having a screen that can be touch to generate an input. As further generally used, the term "projector" refers to an optical device for enlarging a two-dimensional original by suitable guidance of light rays.

In a preferred embodiment, the apparatus may further comprise at least one connecting interface configured for transmitting and/or receiving the first information and/or the second information. In a preferred embodiment, the connecting interface may be at least one of:
- a network interface controller; or
- a transmitter.

As generally used, the term "network interface controller" refers to a computer hardware component that connects a computer to a computer network. As further generally used, the term "transmitter" refers to an electronic device, which produces electromagnetic waves by using an antenna.

In a further aspect, the present invention relates to a use of a personal computer in a method according to the computer-implemented method for determining at least one lens shape for producing at least one optical lens for at least one eye of a person or any preferred embodiment as described elsewhere herein, wherein the personal computer is configured for recording data comprising at least one piece of the second information. In a preferred embodiment, the at least one personal computer may be selected from at least one of:
- a smartphone;
- a smart watch;
- a desktop computer;
- a laptop; or
- a tablet,
wherein the smartphone may, particularly, be preferred, specifically owing to both a high availability in practice and a high practical suitability for the present invention.

With respect to the prior art, the device exhibits the following advantages.

Recording of pieces of second information mostly require an ophthalmologist or optometry specialist. Therefore such test result in reduced portability and cannot be performed by any person itself. As the present invention relies on recording the second information using a personal computer, the recording may be performed anywhere and by any person. In this manner, individual data for a lens prescription may be generated.

As a personal computer, particularly a mobile device is used to determine the at least one piece of second information and a personal computer, respectively a mobile device is available to a large number of persons, the need to buy a separate device is minimized.

As the recorded data can be accessed anywhere by any person having access rights, in particular the person, an optician, an ophthalmologist, or an optic designer, the data can be maintained independent from a specific location. It is further possible to update the data in a convenient manner. This further allows that the person and the expert, in particular the optician, the ophthalmologist, or the optic designer, do not have to be at the same location, such that recommendations can be provided independently from a specific location. In addition, recommendations may be transmitted directly in a fast, convenient and reliable fashion to an optical lens manufacturer.

As used herein, the terms "have", "comprise" or "include" or any arbitrary grammatical variation thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Summarizing, the following Embodiments are particularly preferred within the scope of the present invention:
Embodiment 1. A computer-implemented method for determining at least one lens shape for producing at least one optical lens for at least one eye of a person, the method comprising the following steps:
   a) receiving first information about at least one refractive error of at least one eye of a person and second information related to a person; and
   b) determining at least one lens shape for at least one optical lens for the at least one eye of a person designated for correcting the at least one refractive error of the at least one eye of the person using the first information, wherein at least one lens shape parameter of the at least one optical lens is adapted to the second information related to the person;
   wherein at least one piece of the second information is determined from data recorded by at least one electronic component of at least one personal computer used by the person.
Embodiment 2. The method according to the preceding embodiment, wherein the second information is different from the first information and influences the at least one lens shape parameter.
Embodiment 3. The method according to the any one of the preceding embodiments, wherein the second information is about at least one of:
   - at least one further body feature of the person, more particularly of the at least one eye of the person;
   - at least one habit of the person, particularly selected from at least one of:
      ∘ at least one habit related to the use of the at least one personal computer, or
      ∘ at least one habit related to at least one of an eye movement or a head movement of the person; or
   - at least one parameter related to the environment at the location of the person.
Embodiment 4. The method according to any one of the preceding embodiments, wherein the at least one personal computer is configured for being used by the person for at least one further purpose aside from recording the at least one piece of the second information, particularly for at least one of:
   - surfing in the internet;
   - making phone calls;
   - text editing; or
   - playing computer games.
Embodiment 5. The method according to any one of the preceding embodiments, wherein producing the at least one optical lens comprises at least one of:
   - designing a geometrical model of at least one optical lens for at least one eye of a person comprising the at least one lens shape parameter;
   - manufacturing the at least one optical lens for the at least one eye of the person comprising the geometrical model of at least one optical lens.
Embodiment 6. The method according to any one of the preceding embodiments, wherein the electronic component is configured for recording data comprising the at least one piece of the second information and is, particularly completely, comprised by the at least one personal computer, and particularly connected to the at least one processing device of the at least one personal computer.
Embodiment 7. The method according to any one of the preceding embodiments, wherein the at least one personal computer is selected from at least one of:
   - a smartphone;
   - a smart watch;
   - a smart glasses;
   - a virtual reality head set;
   - a desktop computer;
   - a laptop; or
   - a tablet.
Embodiment 8. The method according to any one of the preceding embodiments, wherein the at least one optical lens is selected from at least one of:
   - a spectacle lens;
   - a contact lens; or
   - an intraocular lens.
Embodiment 9. The method according to any one of the preceding embodiments, wherein the at least one spectacle lens is selected from:
   - a single vision lens;
   - a bifocal lens;
   - a multifocal lens;
   - a variofocal lens; or
   - a progressive-power lens.
Embodiment 10. The method according to any one of the preceding embodiments, wherein the at least one refractive error of the at least one eye of the person is at least one of a value related to:
   - a spherical power;
   - a cylinder;
   - a cylinder axis;
   - a prism;
   - a prism base setting; or
   - an addition.
Embodiment 11. The method according to any one of the preceding embodiments, wherein the at least one refractive error of the at least one eye of the person is received from at least one of:
   - determining by using the at least one personal computer;
   - determining by using at least one lensometer;
   - determining by using at least one autorefractor;
   - determining by using at least one retinoscope;
   - reading in at least one optical lens prescription document, particularly of a spectacle lens; or
   - determining by using at least one subjective refraction workflow.
Embodiment 12. The method according to any one of the preceding Embodiments, wherein the second information comprises at least one piece of second information selected from at least one of:
   - at least one pupil size of the at least one eye of the person; or
   - at least one direction of a line of sight of the at least one eye of the person;
   - at least one angle between a primary direction and the line of sight of the person;
   - at least one orientation of the head of the person;
   - at least one angle of inclination of at least one screen of the at least one personal computer used by the person, particularly with respect to the primary direction;
   - at least one distance between the at least one eye of the person and at least one object located at line of sight of the person;
   - at least one eye movement characteristics of the person;
   - at least one position of a location of the person;
   - at least one screen time of the person;
   - at least one ambient light level at the location of the person, particularly at least one ambient light level when the person is using the at least one personal computer;
   - at least one time ratio of the person being indoors or outdoors;
   - at least one electromagnetic spectrum prevailing at the location of the person;
   - at least one ambient light level at the location of the person; or
   - at least one ultraviolet index at the location of the person.
Embodiment 13. The method according to any one of the preceding Embodiments, wherein the at least one piece of the second information, particularly a plurality of pieces of the second information, recorded in a predefined time interval is assigned to an event. Embodiment 14. The method according to any one of preceding Embodiments, wherein the at least one electronic component of at least one personal computer is selected from least one of:
   - at least one image capturing device;
   - at least one geopositioning sensor;
   - at least one distance sensor;
   - at least one depth sensor;
   - at least one ambient light level sensor;
   - at least one screen inclination sensor; or
   - at least one storage device.
Embodiment 15. The method according to any one of the preceding Embodiments, wherein the at least one image capturing device is selected from at least one of:
   - at least one camera; particularly a front camera and/or back camera of a smartphone;
   - at least one smart sensor, particularly as a combination of a plurality of smart sensors; or
   - at least one webcam.
Embodiment 16. The method according to any one of the preceding Embodiments, wherein the at least one geopositioning sensor may be a tracking unit of at least one of:
   - the Global Positioning System;
   - the Galileo Satellite Navigation;
   - the Global Navigation Satellite System;
   - the BeiDou Navigation Satellite System; or
   - the Indian Regional Navigation Satellite System.
Embodiment 17. The method according to any one of the preceding Embodiments, wherein the at least one storage device is selected from least one of:
   - at least one hard disk;
   - at least one solid state drive; or
   - at least one random access memory.
Embodiment 18. The method according to any one of the preceding Embodiments, wherein the at least one screen inclination sensor is selected from at least one of:
   - at least one accelerometer; or
   - at least one gyroscope.
Embodiment 19. The method according to any one of the preceding Embodiments, wherein the piece of the second information received from data recorded by the at least one image capturing device comprises at least one of:
   - the at least one ambient light level at the location of the person;
   - the at least one electromagnetic spectrum prevailing at the location of the person;
   - the at least one direction of a line of sight of the at least one eye of the person;
   - the at least one orientation of the head of the person;
   - the at least one eye movement characteristics of the person;
   - at least one ultraviolet index at the location of the person; or
   - at least one infrared index at the location of the person;
   - the at least one angle between a primary direction and the line of sight of the person;
   - at least one distance between the at least one eye of the person and at least one object located at line of sight of the person; or
   - the at least one pupil size of the at least one eye of the person.
Embodiment 20. The method according to any one of the preceding Embodiments, wherein the at least one image capturing device is operated at at least one point of time that is unknown to the person and/or at at least one point of time that is known to the at least one person.
Embodiment 21. The method according to any one of the preceding Embodiments, wherein the data recorded by the at least one image capturing device is an image of the person that shows the person in a natural habitus.
Embodiment 22. The method according to any one of the preceding Embodiments, wherein the data recorded by the at least one image capturing device is at least one image of the person that shows the person in a predefined habitus.
Embodiment 23. The method according to any one of the preceding Embodiments, wherein at least one instruction may be given to the person, particularly by displaying the at least one instruction on a screen of the at least one personal computer, on how to adopt the predefined habitus.
Embodiment 24. The method according to any one of the preceding Embodiments, wherein the data recorded by the at least one image capturing device is an image of the person that shows the person wearing spectacles or not wearing spectacles
Embodiment 25. The method according to any one of the preceding Embodiments, wherein the data recorded by the at least one image capturing device is an image of the person processed by using at least one of:
   - at least one image processing method; or
   - at least one machine learning based method.
Embodiment 26. The method according to any one of the preceding Embodiments, wherein the piece of second information received from data recorded by the at least one geopositioning sensor comprises at least one item of information about at least one position of the location of the person, particularly an indication about the at least one person being indoors or outdoors.
Embodiment 27. The method according to any one of the preceding Embodiments, wherein the piece of second information received from data recorded by the at least one storage device is a personal preference of the person using the at least one personal computer, particularly a personal preference of the person using the at least one personal computer selected from at least of:
   - at least one brightness setting of the at least one screen of the at least one personal computer;
   - at least one font size setting of the at least one person using the at least one personal computer; or
   - at least one screen time of the at least one person using the at least one personal computer.
Embodiment 28. The method according to any one of the preceding Embodiments, wherein the at least one font size setting corresponds to a setting of at least of:
   - at least one web browser used by the person; or
   - at least one word processor program used by the person.
Embodiment 29. The method according to any one of the preceding Embodiments, wherein the piece of second information received from data recorded by the screen inclination sensor comprises at least one angle of screen inclination of the at least one screen of the at least one personal computer of the at least one person, particularly with respect to the primary direction.
Embodiment 30. The method according to any one of the preceding Embodiments, wherein data recorded by at least one electronic component is recorded in an acquisition process, wherein the acquisition process comprises at least one of
   - only one single event; or
   - a plurality of events.
Embodiment 31. The method according to any one of the preceding Embodiments, wherein an event comprises a time stamp, wherein a maximal difference in the time stamps of the plurality of events is selected from at least one of:
   - 1, 2, 3, 4, 5, 6, 7, 8, 9, 10, 11, 12, 13, 14, 15, 16, 17, 18, 19, 20, 21, 22, or 23 hours;
   - 1, 2, 3, 4, 5, or 6 days;
   - 1, 2 or 3 weeks; or
   - 1, 2, 3, 4, 5, 6, 7, 8, 9, 10, 11 or 12 months.
Embodiment 32. The method according to any one of the preceding Embodiments, wherein an event comprises a time stamp, wherein the time stamps of the plurality of events are continuously distributed.
Embodiment 33. The method according to any one of the preceding Embodiments, wherein a user, particularly the person, is authenticated, particularly authenticated before the acquisition process is started.
Embodiment 34. The method according to any one of the preceding Embodiments, wherein the person is authenticated by at least one of:
   - a face recognition procedure;
   - a fingerprint analysis procedure; or
   - a login procedure, particularly by requesting at least one of
      ∘ a login;
      ∘ a password; or
      ∘ a pattern.
Embodiment 35. The method according to any one of the preceding Embodiments, wherein the user is selected from at least one of:
   - an optician;
   - an ophthalmologist;
   - an optic designer; or
   - an insurance agent or representative.
Embodiment 36. The method according to any one of the preceding Embodiments, wherein the acquisition process is implemented as a background process.
Embodiment 37. The method according to any one of the preceding Embodiments, wherein the second information is complemented or modified by receiving at least one of:
   - personal data of the person, particularly at least one of:
      ∘ an age;
      ∘ a gender;
      ∘ an ethnicity; or
      ∘ a profession;
   - at least one item of personal preference of the person for the at least one lens shape parameter and/or a recommendation of the optician for the at least one lens shape parameter; or
   - at least one piece of the second information, particularly one piece of the second information that is not recorded by the at least one electronic component of at least one personal computer used by the person.
Embodiment 38. The method according to any one of the preceding Embodiments, wherein the first information and/or the second information is at least one of:
   - complemented or modified; or
   - examined
      by using at least one of:
   - an user interface;
   - a cloud interface; or
   - a connecting interface.
Embodiment 39. The method according to any one of the preceding Embodiments, wherein the user interface and/or the cloud interface is selected from at least one of:
   - a mobile application;
   - a desktop application; or
   - a web application.
Embodiment 40. The method according to any one of the preceding Embodiments, wherein the user interface and/or the cloud interface can be accessed by at least one of:
   - the person;
   - the optician; or
   - an optical lens manufacturer;
   - the optic designer; or
   - the insurance representative.
Embodiment 41. The method according to any one of the preceding Embodiments, wherein the first information and/or the second information is transferred, particularly electronically, to at least one of:
   - a further at least one personal computer;
   - a cloud server;
   - an optician;
   - the ophthalmologist;
   - the optic designer;
   - the insurance agent or representative; or
   - the optical lens manufacturer.
Embodiment 42. The method according to any one of the preceding Embodiments, wherein the optician complements or modifies the first information and/or the second information; and/or examines the first information and/or the second information for giving a recommendation for the at least one lens shape parameter.
Embodiment 43. The method according to any one of the preceding Embodiments, wherein the optical lens manufacturer complements or modifies the first information and/or the second information; and/or examines the first information and/or the second information for giving a recommendation for the at least one lens shape parameter and/or provides a lens shape determining procedure used in determining the at least one lens shape comprising the at least one lens shape parameter.
Embodiment 44. The method according to any one of the preceding Embodiments, wherein a lens shape determining procedure is used in determining the at least one lens shape from at least the first information and the second information, wherein the at least one lens shape comprises the at least one lens shape parameter.
Embodiment 45. The method according to any one of the preceding Embodiments, wherein the lens shape determining procedure considers a third information for determining the at least one lens shape, particularly wherein the third information comprises at least one piece of information selected from at least one of:
   - at least one statistical parameter;
   - at least one parameter determined by using a machine learning model, particularly selected from at least one of:
      ∘ a facial shape parameter;
      ∘ a centration parameter;
      ∘ a fitting parameter; or
      ∘ a further lens shape parameter.
Embodiment 46. The method according to any one of the preceding Embodiments, wherein the at least one centration parameter is selected from at least one of:
   - an interpupillary distance;
   - a vertex distance;
   - a wear pantoscopic angle of the spectacle frame; or
   - a face form angle.
Embodiment 47. The method according to any one of the preceding Embodiments, wherein the at least one fitting parameter may, preferably, be selected from at least one of:
   - a fitting point;
   - a minimum fitting height; or
   - a fitting point mounting position.
Embodiment 48. The method according to any one of the preceding Embodiments, wherein the lens model determining procedure is selected from at least one of:
   - at least one machine learning model;
   - at least one statistical model; or
   - at least one expert knowledge.
Embodiment 49. The method according to any one of the preceding Embodiments, wherein the at least one lens shape parameter of at least one optical lens is at least one of:
   - a magnitude of at least one optical parameter;
   - a distribution of the at least one optical parameter;
   - a gradient of the at least one optical parameter;
   - at least one progression profile of the at least one optical lens;
   - at least one property of at least one viewing zone of the at least one optical lens; particularly
      ∘ at least one property of a viewing zone having a first surface power designed for providing a first refracting power for distance vision; or
      ∘ at least one property of a viewing zone having a second surface power designed for providing a second refractive power corresponding to near vision; or
      ∘ at least one property of a viewing zone having a third surface power designed for providing a third refracting power for a different type of vision;
   - a material of the at least one optical lens;
   - a filter of the at least one optical lens;
   - a color tint of the at least one optical lens; or
   - a refractive index of the material of the at least one optical lens.
Embodiment 50. The method according to any one of the preceding Embodiments, wherein the at least one optical parameter may be at least one of:
   - a spherical power; or
   - a cylinder;
   - a cylinder axis;
   - a prismatic power;
   - a prism base setting; or
   - an addition.
Embodiment 51. The method according to any one of the preceding Embodiments, wherein the progression profile is described by at least one parameter, wherein the at least one parameter is selected from at least one of:
   - a spatial position of at least one progression start on a surface of the at least one optical lens;
   - a spatial position of at least one progression end on the surface of the at least one optical lens;
   - a distance between the spatial position of the at least one progression start and the spatial position of the at least one corresponding progression end on the surface of the at least one optical lens;
   - a course of the progression from the progression start to the progression end; or
   - a width of a progression corridor.
Embodiment 52. The method according to any one of the preceding Embodiments, wherein the at least at least one progression profile is, in addition to the at least one refractive error of the at least one eye of the person, adapted to at least one of:
   - the age of the person;
   - the at least one angle of inclination of a screen of the at least one personal computer;
   - a zone demand profile considering different viewing zones of the person;
   - a head and/or eye movement profile of the person; or
   - an activity profile of the person.
Embodiment 53. The method according to any one of the preceding Embodiments, wherein the activity profile of the person is determined from at least one piece of the second information and/or received from at least one of: the user interface, the cloud interface, or the connecting interface.
Embodiment 54. The method according to any one of the preceding Embodiments, wherein the at least one property of the at least one viewing zone,
   particularly wherein the at least one viewing zone is selected from at least one of:
   ∘ a viewing zone for distance vision;
   ∘ a viewing zone for near vision; or
   ∘ a viewing zone for intermediate vision;
   is adapted to at least one of:
   - the at least one pupil size of the at least one eye of the person, particularly the at least one pupil size of the at least one eye of the person at a given at least one of:
      ∘ at least one ambient light level;
      ∘ the at least one distance between the at least one eye of the person and at least one object located at line of sight of the person; or
      ∘ the activity profile of the person; or
   - the at least one distance between the at least one eye of the person and at least one intersection point of the line of sight of the person and the screen of the at least one personal computer.
Embodiment 55. The method according to any one of the preceding Embodiments, wherein the at least one property of the viewing zone for distance vision is adapted to at least one of:
   - the at least one pupil size of the at least one eye of the person, particularly the at least one pupil size of the at least one eye of the person at a given at least one ambient light level; or
   - the at least one pupil size of the at least one eye of the person, particularly the at least one pupil size of the at least one eye of the person at a given at least one viewing distance; or
   - the at least one pupil size of the at least one eye of the person, particularly the at least one pupil size of the at least one eye of the person at a given at least one activity.
Embodiment 56. The method according to any one of the preceding Embodiments, wherein the at least one property of the viewing zone for near vision is adapted to at least one of:
   - the at least one pupil size of the at least one eye of the person, particularly the at least one pupil size of the at least one eye of the person at a given at least one ambient light level; or
   - the at least one distance between the at least one eye of the person and at least one intersection point of the line of sight of the person and the screen of the at least one personal computer.
Embodiment 57. The method according to any one of the preceding Embodiments, the at least one property of the viewing zone for intermediate vision is adapted to at least one of:
   - the at least one pupil size of the at least one eye of the person, particularly the at least one pupil size of the at least one eye of the person at a given at least one ambient light level; or
   - the at least one distance between the at least one eye of the person and at least one intersection point of the line of sight of the person and the screen of the at least one personal computer.
Embodiment 58. The method according to any one of the preceding Embodiments, wherein
   - a dioptric power of the at least one optical lens, particularly the spectacle lens, is determined from the first information; and
   - a distribution of a surface power over a surface of the at least one optical lens, particularly the spectacle lens, is adapted to the at least one piece of second information.
Embodiment 59. The method according to any one of the preceding Embodiments, wherein a distribution of a surface power over a surface of the at least one optical lens, particularly the spectacle lens, is adapted to at least one of:
   - the at least one screen time of the at least one person using the at least one personal computer; or
   - at least one outdoor time of the at least one person spent outdoors.
Embodiment 60. A computer program comprising instructions which, when the program is executed by a computer, cause the computer to carry out a method according to any one of the preceding method Embodiments.
Embodiment 61. An apparatus for determining at least one lens shape for producing at least one optical lens for at least one eye of a person, the apparatus comprising at least one processing device configured for
   ∘ receiving first information about at least one refractive error of at least one eye of a person and second information related to a person; and
   ∘ determining at least one lens shape for least one optical lens for the at least one eye of a person designated for correcting the at least one refractive error of the at least one eye of the person using the first information, wherein at least one lens shape parameter of the at least one optical lens is adapted to the second information related to the person;
   wherein the at least one processing device is further configured for determining at least one piece of the second information from data recorded by at least one electronic component of at least one personal computer used by the person.
Embodiment 62. The apparatus according to the preceding Embodiment, wherein the at least one processing device is further configured for carrying out any of the preceding method Embodiments.
Embodiment 63. The apparatus according to any one of the preceding apparatus Embodiments, wherein the apparatus is the at least one personal computer.
Embodiment 64. The apparatus according to any one of the preceding apparatus Embodiments, wherein the apparatus is at least one of:
   - a smartphone;
   - a smart watch;
   - a smart glasses;
   - a virtual head set;
   - a desktop computer;
   - a laptop; or
   - a tablet.
Embodiment 65. The apparatus according to any one of the preceding apparatus Embodiments, wherein the apparatus further comprises at least one image capturing device as electronic component configured for recording the at least one piece of the second information.
Embodiment 66. The apparatus according to any one of the preceding apparatus Embodiments, wherein the at least one image capturing device is at least one of:
   - a camera; particularly a front camera and/or back camera of a smartphone; or
   - a webcam.
Embodiment 67. The apparatus according to any one of the preceding apparatus Embodiments, wherein the apparatus further comprises at least one geopositioning sensor as electronic component configured for recording the at least one piece of the second information.
Embodiment 68. The apparatus according to any one of the preceding apparatus Embodiments, wherein the at least one geopositioning sensor may be a tracking unit of at least one of:
   - the Global Positioning System;
   - the Galileo Satellite Navigation;
   - the Global Navigation Satellite System;
   - the BeiDou Navigation Satellite System; or
   - the Indian Regional Navigation Satellite System.
Embodiment 69. The apparatus according to any one of the preceding apparatus Embodiments, wherein the apparatus further comprises at least one screen inclination sensor as electronic component configured for recording the at least one piece of the second information.
Embodiment 70. The apparatus according to any one of the preceding apparatus Embodiments, wherein the at least one screen inclination sensor is at least one of:
   - an accelerometer; or
   - a gyroscope.
Embodiment 71. The apparatus according to any one of the preceding apparatus Embodiments, wherein the apparatus further comprises at least one storage device as electronic component configured for storing the at least one piece of the second information.
Embodiment 72. The apparatus according to any one of the preceding apparatus Embodiments, wherein the at least one storage device being at least one of:
   - a hard disk;
   - at least one solid state drive; or
   - a random access memory.
Embodiment 73. The apparatus according to any one of the preceding apparatus Embodiments, the apparatus further comprising at least one screen configured for displaying a user interface.
Embodiment 74. The apparatus according to any one of the apparatus preceding Embodiments, wherein the at least one screen is at least one of:
   - a touchscreen;
   - a monitor; or
   - a projector.
Embodiment 75. The apparatus according to any one of the preceding apparatus Embodiments, the apparatus further comprising at least one connecting interface configured for transmitting and/or receiving the first information and/or the second information.
Embodiment 76. The apparatus according to any one of the preceding apparatus Embodiments, wherein the connecting interface is at least one of:
   - a network interface controller; or
   - a transmitter.
Embodiment 77. A use of a personal computer in a method according the any of the preceding method Embodiments, wherein the personal computer is configured for recording data comprising at least one piece of the second information.
Embodiment 78. A use of an personal computer according to any one of the preceding use Embodiments, wherein the at least one personal computer is selected from at least one of:
   - a smartphone;
   - a smart glasses;
   - a virtual head set;
   - a desktop computer;
   - a laptop; or
   - a tablet.

### Short description of the Figures

Further optional features and embodiments of the present invention are disclosed in more detail in the subsequent description of preferred embodiments, preferably in conjunction with the dependent claims. Therein, the respective optional features may be realized in an isolated fashion as well as in any arbitrary feasible combination, as the skilled person will realize. It is emphasized here that the scope of the invention is not restricted by the preferred embodiments.

In the Figures:
- Figure 1: illustrates an aerial view of an exemplary apparatus for determining at least one lens shape;
- Figure 2: illustrates a schematic view of a computer program running on the exemplary apparatus for carrying out a method for determining the at least one lens shape;
- Figure 3: illustrates an exemplary embodiment of a contour plot of a surface mean power (Fig. 3a) and a corresponding progression profile (Fig. 3b) of a determined lens shape;
- Figure 4: illustrates a further exemplary embodiment of a contour plot of a surface mean power (Fig. 4a) and a corresponding progression profile (Fig. 4b) of a further determined lens shape;
- Figure 5: illustrates two further exemplary embodiments of a contour plot of a surface mean power (Figs. 5a and 5b) of a further determined lens shape;
- Figure 6: illustrates a side view of an eye of a person directed at different viewing zones.

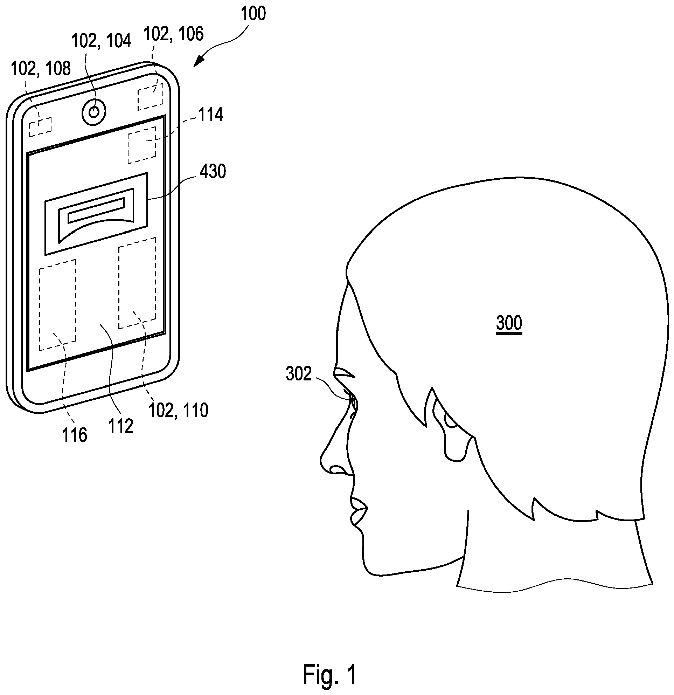

Figure 1 shows an aerial view of an exemplary apparatus 100 for determining at least one lens shape 202 for producing an optical lens 200 for at least one eye 302 of a person 300. The apparatus 100 comprises a processing device 114. Any component, such as the processing device 114, which is illustrated here by using dashed lines is located within the apparatus 100. The processing device 114 is configured for receiving first information about at least one refractive error of at least one eye 302 of a person 300. The processing device 114 is further configured for receiving second information related to the person 300.

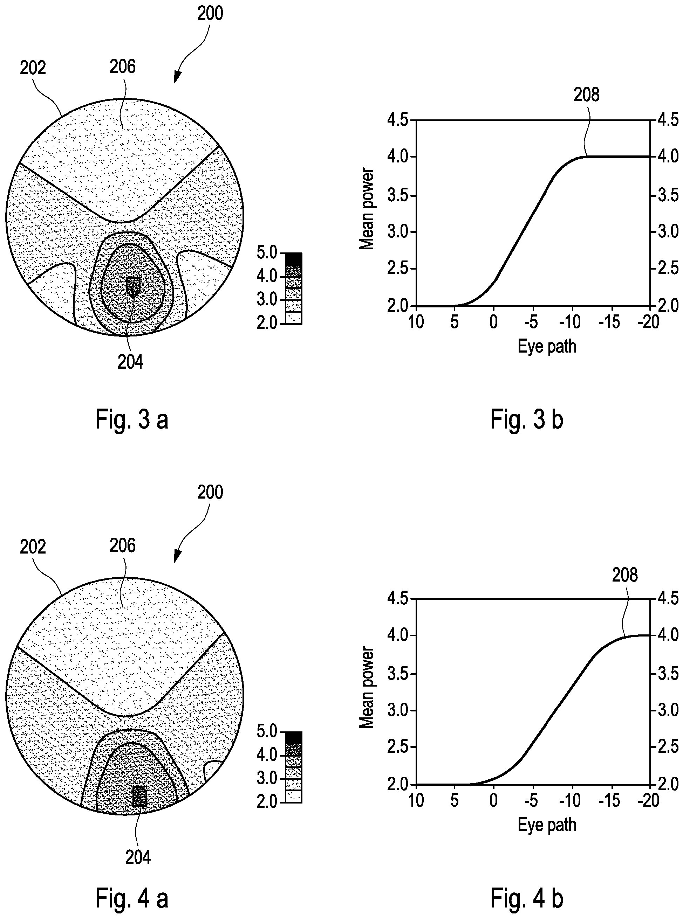

The first information and the second information are used in determining a lens shape 202 for an optical lens 200 for the eye 302 of a person 300. Figure 3 illustrates an exemplary embodiment of a contour plot related to the determined lens shape 202 of the optical lens 200 being a spectacle lens. Alternatively, the optical lens 200 may be a contact lens or an intraocular lens. The optical lens 200 is designated for correcting the at least one refractive error of the at least one eye 302 of the person 300. The lens shape 202 comprises at least one lens shape parameter adapted to the second information related to the person 300. At least one piece of the second information is determined from data recorded by at least one electronic component 102 of at least one personal computer used by the person 300. The apparatus 100 is the at least one personal computer.

In the exemplary apparatus 100 as depicted in Figure 1, the at least one personal computer is a smartphone, particularly owing to both a high availability in practice and a high practical suitability for the present invention. However, as an alternative, the at least one personal computer may be selected from a desktop computer, a laptop, a tablet, a smart glasses, a virtual reality head set or a smart watch.

The apparatus 100 as illustrated in Figure 1 further comprises an image capturing device 104 as electronic component 102 configured for recording the at least one piece of the second information. The image capturing device 104 as used herein is a camera, specifically a front camera of the smartphone. Alternatively, the image capturing device 104 may be a back camera of the smartphone, a webcam, or a smart sensor. The image capturing device 104 is further used herein as a distance sensor and an ambient light level sensor.

The apparatus 100 as shown in Figure 1 further comprises a geopositioning sensor 106 as electronic component 102 configured for recording the at least one piece of the second information. The geopositioning sensor 106 is a tracking unit of the Global Positioning System. Alternatively, the geopositioning sensor 106 may be a tracking unit of the Galileo Satellite Navigation, the Global Navigation Satellite System, the BeiDou Navigation Satellite System, or the Indian Regional Navigation Satellite System.

The apparatus 100 as depicted in Figure 1 further comprises a screen inclination sensor 108 as electronic component 102 configured for recording the at least one piece of the second information. The screen inclination sensor 108 is a combination of an accelerometer and a gyroscope.

The apparatus 100 as illustrated shown in Figure 1 further comprises a storage device 110 as electronic component 102 configured for storing the at least one piece of the second information. The storage device 110 is solid state drive. Alternatively, the storage device 110 may be a hard disk or a random access memory.

The apparatus 100 as shown in Figure 1 further comprises a screen 112 which is configured for displaying a user interface 430. The screen 112 as used herein is a touchscreen; as an alternative, the screen 112 may be a monitor, or a projector.

The apparatus 100 as depicted in Figure 1 further comprises a connecting interface 116 configured for transmitting and receiving the first information and the second information. The connecting interface 116 is a transmitter. Alternatively, the connecting interface 116 may be a network interface controller.

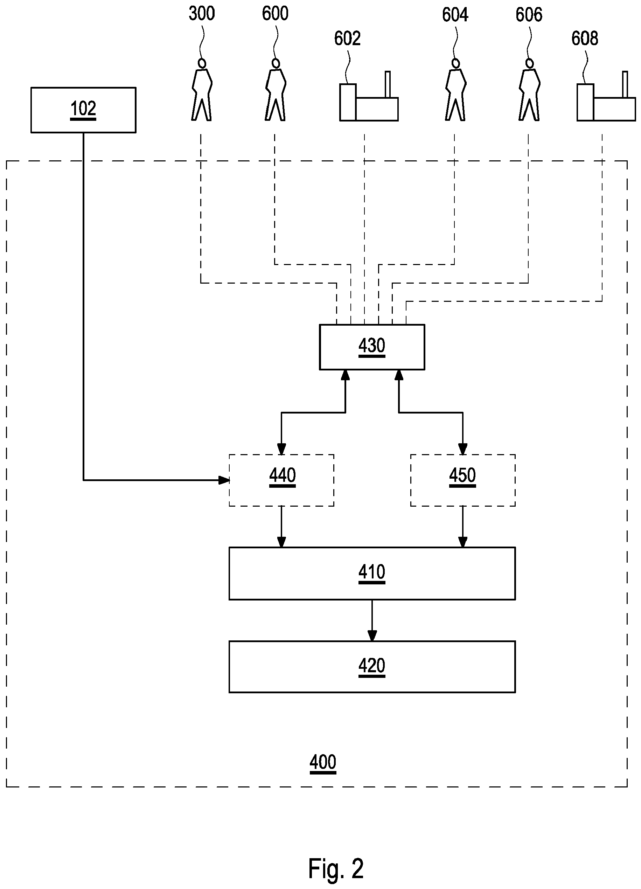

A computer-implemented method 400 for determining at least one lens shape 202 for producing an optical lens 200 for the eye 302 of the person 300 which may, preferably, be performed by using the apparatus 100 is schematically displayed in Figure 2.

In a receiving step 410, first information 450 about the refractive error of the eye 302 of the person 300 and second information 440 related to the person 300 are received. Herein, the first information 450 may comprise at least one value related to the refractive error of the eye 302 of the person 300, in particular a value related to a spherical power and, if applicable, a cylinder and a cylinder axis. Further, the first information 450 may, additionally, comprise values related to a prism, a prism base setting, or an addition to be used for correcting the refractive error of the eye 302 of the person 300.

Herein, the at least one value related to the refractive error of the at least one eye 302 of the person 300 may be received from reading at least one optical lens prescription document, particularly of the spectacle lens. Alternatively, the at least one value related to the refractive error of the at least one eye 302 of the person 300 may be determined by using the apparatus 100, especially by using a lensometer, an autorefractor and/or retinoscope or by using at least one subjective refraction workflow designated for such a purpose.

The piece of the second information 440 received from data recorded by the electronic component 102, especially by using the image capturing device 104, may, preferably, comprise a direction of a line of sight of the at least one eye 302 of the person 300 intersecting with the screen 112 of the apparatus 100. In addition, the activity which is performed by the person 300 at that moment can be identified as reading. A further piece of second information 440 which can be determined from data recorded by the screen inclination sensor 108 may comprise an angle of screen inclination of the screen 112 of the apparatus 100.

The data recorded by the at least one image capturing device 104 is an image of the person 300 that shows the person 300 in a natural habitus during reading. For this purpose, the image may be captured at a point of time which is known to the person 300. However, the image capturing device 104 may, preferably, be operated at a point of time which is unknown to the person 300, especially in order to minimize a conscious influence of the person 300 on a related acquisition process. Herein, it may, advantageously, be possible to implement the acquisition process as a background process. Alternatively or in addition, the data recorded by the at least one image capturing device 104 is at least one image of the person 300 that shows the person 300 in a predefined habitus. At least one instruction may be given to the person 300, particularly by displaying the at least one instruction on the screen 112 of the at least one personal computer, on how to adopt the predefined habitus.

During the acquisition process is running the person 300 is may be using a web interface 430. The image of the person 300 shows that the person 300 is not wearing spectacles. Alternatively, the image may show that the person 300 may be wearing spectacles. To receive a relevant piece of the second information 440, an image processing method can be applied. Alternatively or in addition, a machine learning based method may be used for such a purpose.

At least one piece of second information 440 is received from the electronic components 102. The piece of the second information 440 received from data recorded by the image capturing device 104 may, further, comprise an ambient light level at the location of the person 300, an electromagnetic spectrum prevailing at the location of the person 300, an angle between a primary direction and the line of sight of the person 300, a distance between the eye 302 of the person 300 and an object located at line of sight of the person 300, or the at least one pupil size of the eye 302 of the person 300, at least one orientation of the head of the person 300, the at least one eye movement characteristics of the person 300, at least one ultraviolet index at the location of the person 300, or at least one infrared index at the location of the person 300.

The piece of second information 440 received from data recorded by the geopositioning sensor 106 may comprise at least one item of information about a position of the location of the person 300, particularly an indication whether the person 300 may stay indoors or outdoors. The piece of second information 440 received from the storage device 110 may be a personal preference of the person 300 using the apparatus 100. The personal preference may be a brightness setting of the screen 112, a font size setting of the person 300, or a screen time of the person 300. Herein, the font size setting may be a setting of a web browser or a word processor program used by the person 300.

The data recorded by at least one electronic component 102 is recorded in an acquisition process, wherein the acquisition process may comprise a plurality of events. Alternatively, it may comprise only a single event. Herein, each event may comprise a time stamp. An exemplary maximal time difference in the time stamps of the plurality of events may be set to 6 days. A different value for the time difference may be possible. In a particular embodiment, the time stamps of the plurality of events may be continuously distributed over the maximal time difference.

The user interface 430 as used herein may be a mobile application; as an alternative, the user interface may be a cloud interface, a connecting interface, or a web application. Especially, the user interface 430 may allow for complementing, modifying or examining the first information 450 and the second information 440.

The user interface 430 may be accessed by the person 300, an optician 600, an optical lens manufacturer 602, an optic designer 604, an insurance representative 606, or an ophthalmologist 608. Therefore, the first information 450 and the second information is transferred between the respective users. Each user 300, 600, 602, 604, 606, 608, particularly the person 300, may be authenticated before logging into the user interface 430. The person 300 is particularly authenticated before the acquisition process is started.

The person 300 as depicted in Figure 1 has been authenticated by a face recognition procedure. Alternatively, the person 300 may be identified by a fingerprint analysis procedure, a login procedure, particularly by requesting a login and a password or an unlock pattern.

Further, the optician 600 may complement or modify the first information 450 and the second information 440. Additionally, the optician 600 may examine the first information 450 and the second information 440 for giving a recommendation for the at least one lens shape parameter.

Alternatively or in addition, the optical lens manufacturer 602 may complement or modify the first information 450 and the second information 440. The optical lens manufacturer 602 may further examine the first information 450 and the second information 440 for giving a recommendation for the at least one lens shape parameter. The optical lens manufacturer 602 may, additionally, provide a lens shape determining procedure used in determining the lens shape 202 comprising the at least one lens shape parameter.

The second information 440 may be complemented or modified by receiving personal data of the person 300, such as an age, a gender, an ethnicity, or a profession. The second information 400 may further be complemented or modified by at least one item of personal preference of the person 300 for the lens shape parameter and a recommendation of the optician 600 for the lens shape parameter. Additionally a piece of the second information that is not recorded by any of the electronic components 102 may be complemented.

In a determining step 420, the lens shape 202 of the optical lens 200 for the eye 302 of the person 300 designated for correcting the refractive error of the eye 302 of the person 300 is determined. For this purpose, the lens shape parameter of optical lens 200 is adapted to the second information 440 related to the person. For such a purpose, a lens shape determining procedure may be used for determining the lens shape 202 from the first information 450 and the second information 440, wherein the lens shape 202 comprises at least one lens shape parameter.

Further, third information comprising a statistical parameter and/or a parameter determined by using a machine learning model, specifically a facial shape parameter, a centration parameter, a fitting parameter or a further lens shape parameter, may be used in determining the lens shape 200. The at least one centration parameter may be selected from, alternatively or in addition, an interpupillary distance, a vertex distance; a wear pantoscopic angle of the spectacle frame or a face form angle. The fitting parameter may, alternatively or in addition, be selected from a fitting point, a minimum fitting height or a fitting point mounting position.

The lens shape determining procedure may be a machine learning model. Alternatively, it may be a statistical model or the knowledge of an expert.

The exemplary optical lens 200 being a spectacle lens for which the lens shape 202 was determined may, as illustrated in Figure 3a, be a progressive-power lens. Alternatively, it may be a single vision lens, a multifocal lens, a variofocal lens, or a progressive-power lens. As illustrated in the contour plot of a surface mean power of the optical lens 200 in Figure 3a, the position of a viewing zone for near vision 204 and the viewing zone for distance vision 206 is adapted to the second information 440, particularly the recorded line of sight and the recorded screen inclination angle while reading. The adapted lens shape parameter of the optical lens 200 is further a progression profile 208 of the optical lens 200, which is depicted in Figure 3b.

Figures 4a and 4b illustrate a further exemplary embodiment of a contour plot of the determined lens shape 202 of the optical lens 200 which is here a bifocal lens. The optical lens 200 as depicted there exhibits the same optical parameters as the optical lens 200 as shown in Figures 3a and 3b. However, the exemplary optical lens 200 as shown in Figures 3a and 3b has been adapted to different pieces of second information 440 compared to the exemplary optical lens 200 as depicted in Figures 4a and 4b, particularly a different line of sight and a different screen inclination angle while reading. As a result, the viewing zone for near vision 204 is positioned at a location closer to the bottom of the optical lens 200 as shown in Figures 4a and 4b. In addition, the progression profile 208 is different.

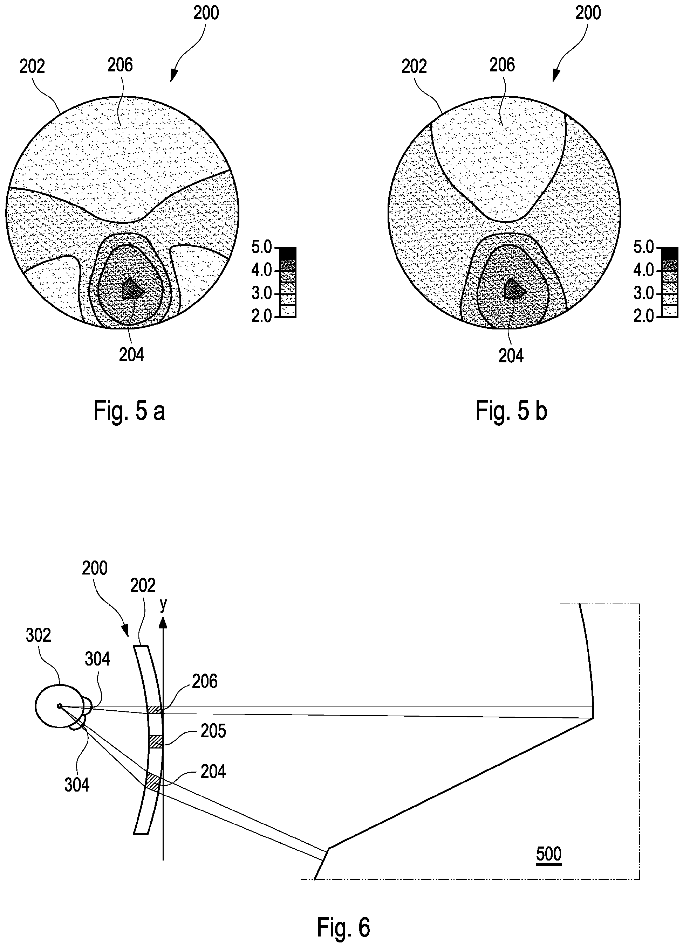

As a further example, a design of the viewing zone for distance vision 206 may also be provided. Figure 5a illustrates a contour plot of a surface mean power of a further exemplary lens shape 202 of the optical lens 200 having a so-denoted "hard design". In contrast hereto, Figure 5b illustrates a further contour plot of the surface mean power of a still further exemplary lens shape 202 of the optical lens 200 having a so-denoted "soft design". The influence of the type of design on area and position of the viewing zone for distance vision 206 can be recognized by comparing Figures 5a and 5b.

Herein, a person 300 who is frequently involved in activities that require a large viewing zone for distance vision 206, such as for activities comprising repeated driving or exercising sport, may prefer the exemplary optical lens 200 as illustrated in Figure 5a having the hard design, especially since this person 300 has a zone demand profile according to which the viewing zone for distance vision 206 may, preferably, have an extended area. Herein, the zone demand profile may be determined by using a head and eye movement profile of the person 300 as well as by a value for the time spent outdoors by the person 300. On the other hand, a person 300 that spends most time indoors and has a large screen time as indicated by the time the apparatus 100 may be used by the person 300 may, especially, prefer the exemplary optical lens 200 as shown in Figure 5b having the soft design.

In other words, a value for a dioptric power of any depicted optical lens 200 may be determined from the first information 450, whereas a distribution of a surface power over a surface of each optical lens 200 may be adapted to at least one piece of the second information 440. Herein, further degrees of freedom for selecting the design may, additionally, be considered. Further, a refractive index of the material of the optical lens 200 may be a further lens shape parameter to be determined. Still further, the progression profile 208 may, be adapted to the age of the person 300. Still further, an area of the viewing zone for distance vision 206 may be adapted to the pupil size of the eye 302 of the person 300 at a given ambient light level, a given viewing distance and/or a given activity.

Figure 6 illustrates a side view of the eye 302 of the person 300 in two positions. In a first position, the eye 302 is looking through the viewing zone for distance vision 206 onto an object 500, while, in a second position, the eye 302 is looking through the viewing zone for near vision 204 onto the object 500, as indicated by respective lines. The viewing zone for near vision 204 is adapted to the at least one pupil size 304 of the eye 302 of the person 300 and to the distance between the eye 300 and the object 500. The viewing zone for distance vision 206 is also adapted to the pupil size 304 of the eye 302 of the person 300 at a given at least one ambient light level, viewing distance and/or activity. Further, a property of a viewing zone having a third surface power designed for providing a third refracting power may, in a similar manner, be adapted, in particular to the second information. The third viewing zone may be an intermediate viewing zone 205 as depicted.

### List of Reference Signs

- 100: apparatus
- 102: electronic component
- 104: image capturing device
- 106: geopositioning sensor
- 108: screen inclination sensor
- 110: storage device
- 112: screen
- 114: processing device
- 116: connecting interface
- 200: optical lens
- 202: lens shape
- 204: viewing zone for near vision
- 205: viewing zone for intermediate vision
- 206: viewing zone for distance vision
- 208: progression profile
- 300: person
- 302: eye
- 304: pupil size
- 400: method for determining at least one lens shape
- 410: receiving step
- 420: determining step
- 430: user interface
- 440: second information
- 450: first information
- 500: object
- 600: optician
- 602: ophthalmologist
- 604: optic designer
- 606: insurance representative
- 608: optical lens manufacturer

## Claims

1. A computer-implemented method (400) for determining at least one lens shape (202) for producing at least one optical lens (200) for at least one eye (302) of a person (300), the method comprising the following steps:
a) receiving first information (450) about at least one refractive error of at least one eye (302) of a person (300) and second information (440) related to the person (300);
b) determining at least one lens shape (202) for at least one optical lens (200) for the at least one eye (302) of the person (300) designated for correcting the at least one refractive error of the at least one eye (302) of the person (300) using the first information (450), wherein at least one lens shape parameter of the at least one optical lens (200) is adapted to the second information (440) related to the person (300);
**characterized in that**
at least one piece of the second information (440) is determined from data recorded by at least one electronic component (102) of at least one personal computer used by the person (300).

2. The method (400) according to the preceding claim, wherein the at least one personal computer is selected from at least one of:
- a smartphone;
- a smart watch;
- a smart glasses;
- a virtual reality head set;
- a desktop computer;
- a laptop; or
- a tablet.

3. The method (400) according to any one of the preceding claims, wherein the at least one electronic component (102) of at least one personal computer is selected from least one of:
- at least one image capturing device (104);
- at least one geopositioning sensor (106);
- at least one distance sensor;
- at least one depth sensor;
- at least one ambient light level sensor;
- at least one screen inclination sensor (108); or
- at least one storage device (110).

4. The method (400) according to the preceding claim, wherein the piece of the second information (440) received from data recorded by the at least one image capturing device (104) comprises at least one of:
- at least one ambient light level at the location of the person (300);
- at least one electromagnetic spectrum prevailing at the location of the person (300);
- at least one direction of a line of sight of the at least one eye (302) of the person (300);
- at least one orientation of the head of the person (302);
- at least one eye movement characteristics of the person (302);
- at least one ultraviolet index at the location of the person (302); or
- at least one infrared index at the location of the person (302);
- at least one angle between a primary direction and the line of sight of the person (300);
- at least one distance between the at least one eye (302) of the person (300) and at least one object located at line of sight of the person (300); or
- at least one pupil size (304) of the at least one eye (302) of the person (300).

5. The method (400) according to any one of the two preceding claims, wherein the piece of second information (440) received from data recorded by the at least one storage device is a personal preference of the person (300) using the at least one personal computer, particularly a personal preference of the person (300) using the at least one personal computer selected from at least of:
- at least one brightness setting of the at least one screen (112) of the at least one personal computer;
- at least one font size setting of the at least one person (300) using the at least one personal computer; or
- at least one screen time of the at least one person (300) using the at least one personal computer.

6. The method (400) according to any one of the preceding claims, wherein the second information (440) is at least one of:
- complemented or modified; or
- examined
by using at least one of:
- an user interface (430);
- a cloud interface; or
- a connecting interface.

7. The method (400) according to any one of the preceding claims, wherein at least one of the first information (450) or the second information (440) is transferred, particularly electronically, to at least one of:
- a further at least one personal computer;
- a cloud server;
- an optician (600); particularly wherein the optician (600)
∘ complements or modifies at least one of the first information (450) or the second information (440); or
∘ examines at least one of the first information (450) or the second information (440) for giving a recommendation for the at least one lens shape parameter;
- an ophthalmologist (602); particularly wherein the ophthalmologist (602)
∘ complements or modifies at least one of the first information (450) or the second information (440); or
∘ examines at least one of the first information (450) or the second information (440) for giving a recommendation for the at least one lens shape parameter;
- an optic designer (604);
- an insurance agent or insurance representative (606); or
- an optical lens manufacturer (608).

8. The method (400) according to any one of the preceding claims, wherein the at least one lens shape parameter of at least one optical lens (200) is at least one of:
- a magnitude of at least one optical parameter;
- a distribution of the at least one optical parameter;
- a gradient of the at least one optical parameter;
- at least one progression profile (208) of the at least one optical lens (200);
- at least one property of at least one viewing zone (204, 206) of the at least one optical lens (200); particularly
∘ at least one property of a viewing zone (204, 206) having a first surface power designed for providing a first refracting power for distance vision; or
∘ at least one property of a viewing zone (204, 206) having a second surface power designed for providing a second refractive power corresponding to near vision; or
∘ at least one property of a viewing zone (204, 206) having a third surface power designed for providing a third refracting power for a different type of vision;
- a material of the at least one optical lens (200);
- a filter of the at least one optical lens (200);
- a color tint of the at least one optical lens (200); or
- a refractive index of the material of the at least one optical lens (200).

9. The method (400) according to the preceding claim, wherein the at least at least one progression profile (208) is, in addition to the at least one refractive error of the at least one eye (302) of the person (300) adapted to at least one of:
- the age of the person (300);
- the at least one angle of inclination of a screen of the at least one personal computer;
- a zone demand profile considering different viewing zones (204, 206) of the person (300);
- a eye (302) movement profile of the person (300); or
- an activity profile of the person (300).

10. The method (400) according to any one of the two preceding claims, wherein the at least one property of the at least one viewing zone (204, 206) is adapted to at least one of:
- the at least one pupil size (304) of the at least one eye (302) of the person (300), particularly the at least one pupil size (304) of the at least one eye (302) of the person (300) at a given at least one of:
∘ at least one ambient light level;
∘ the at least one distance between the at least one eye of the person and at least one object located at line of sight of the person; or
∘ the activity profile of the person; or
- the at least one distance between the at least one eye (302) of the person (300) and at least one intersection point of the line of sight of the person (300) and the screen of the at least one personal computer.

11. The method (400) according to any one of the preceding claims, wherein a distribution of a surface power over a surface of the at least one optical lens (200) is adapted to:
- the at least one screen time of the at least one person (300) using the at least one personal computer;
- at least one outdoor time of the at least one person (300) spent outdoors.

12. The method (400) according to any one of the preceding claims, wherein
- a dioptric power of the at least one optical lens (200) is determined from the first information (450); and
- a distribution of a surface power over a surface of the at least one optical lens (200) is adapted to at least one piece of second information (440).

13. A computer program comprising instructions which, when the program is executed by a computer, cause the computer to carry out a computer-implemented method for determining at least one lens shape (202) for producing at least one optical lens (200) for at least one eye (302) of a person (300), the method (400) comprising the following steps:
a) receiving first information (450) about at least one refractive error of at least one eye (302) of a person (300) and second information (440) related to the person (300);
b) determining at least one lens shape (202) for at least one optical lens (200) for the at least one eye (302) of the person (300) designated for correcting the at least one refractive error of the at least one eye (302) of the person (300) using the first information (450), wherein at least one lens shape parameter of the at least one optical lens (200) is adapted to the second information (440) related to the person (300);
**characterized in that**
at least one piece of the second information (440) is determined from data recorded by at least one electronic component (102) of at least one personal computer used by the person (300).

14. An apparatus (100) for determining at least one lens shape (202) for producing at least one optical lens (200) for at least one eye (302) of a person (300), the apparatus (100) comprising at least one processing device (104) configured for
∘ receiving first information (450) about at least one refractive error of at least one eye (302) of a person (300) and second information (440) related to the person (300);
∘ determining at least one lens shape (202) for least one optical lens (200) for the at least one eye (302) of the person (300) designated for correcting the at least one refractive error of the at least one eye (302) of the person (300) using the first information (450), wherein at least one lens shape parameter of the at least one optical lens (200) is adapted to the second information (440) related to the person (300);
**characterized in**
**that** the at least one processing device (104) is further configured for determining at least one piece of the second information (440) from data recorded by at least one electronic component (102) of at least one personal computer used by the person (300).

15. A use of a personal computer in a method for determining at least one lens shape (202) for producing at least one optical lens (200) for at least one eye (302) of a person (300), the method (400) comprising the following steps:
a) receiving first information (450) about at least one refractive error of at least one eye (302) of a person (300) and second information (440) related to the person (300);
b) determining at least one lens shape (202) for at least one optical lens (200) for the at least one eye (302) of the person (300) designated for correcting the at least one refractive error of the at least one eye (302) of the person (300) using the first information (450), wherein at least one lens shape parameter of the at least one optical lens (200) is adapted to the second information (440) related to the person (300);
**characterized in**
**that** at least one piece of the second information (440) is determined from data recorded by at least one electronic component (102) of the at least one personal computer used by the person (300).
